# EUROPEAN PATENT SPECIFICATION

(11) **EP 4 351 582 B1**
(45) Date of publication and mention of the grant of the patent: **08.10.2025**
(21) Application number: 22733341.6
(22) Date of filing: 07.06.2022
(51) Int. Cl.: A61K 31/517, A61P 35/00, A61K 45/06, A61K 39/395, C07K 16/28

(54) **COMBINATION OF A PARTICULAR BRAF INHIBITOR (PARADOX BREAKER) AND A PD-1 AXIS BINDING ANTAGONIST FOR USE IN THE TREATMENT OF CANCER**
KOMBINATION EINES BESTIMMTEN BRAF-INHIBITORS (PARADOXBRECHER) MIT EINEM PD-1 ACHSENBINDENDEN ANTAGONISTEN ZUR VERWENDUNG IN DER KREBSBEHANDLUNG
COMBINAISON D'UN INHIBITEUR PARTICULIER DE BRAF (BRISEUR DE PARADOXE) AVEC UN ANTAGONISTE SE LIANT À L'AXE PD-1 POUR LE TRAITEMENT DU CANCER

(30) Priority: 09.06.2021 EP 21178462; 18.02.2022 EP 22157499
(43) Date of publication of application: 17.04.2024
(73) Proprietor: F. Hoffmann-La Roche AG, 4070 Basel (CH)
(72) Inventor: ECKMANN, Jan, 82377 Penzberg (DE); FRIESS, Thomas, 82377 Penzberg (DE); HERTING, Frank, 82377 Penzberg (DE); PETTAZZONI, Piergiorgio Francesco Tommaso, 4070 Basel (CH); WICHMANN, Juergen, 4070 Basel (CH)
(74) Representative: Vitra, Hermeto
(86) International application number: PCT/EP2022/065373
(87) International publication number: WO 2022/258600

(56) References cited:
- WO-A1-2018/075447
- WO-A1-2021/116055

## Description

The present invention relates to a combination of a BRAF inhibitor of formula (I) or a pharmaceutically acceptable salt or solvate thereof, and a PD-1 axis binding antagonist, as well as uses and pharmaceutical compositions thereof.

The present invention provides in particular a BRAF inhibitor and a PD-1 axis binding antagonist for use in the treatment of cancer, wherein the BRAF inhibitor is a compound of formula (I) 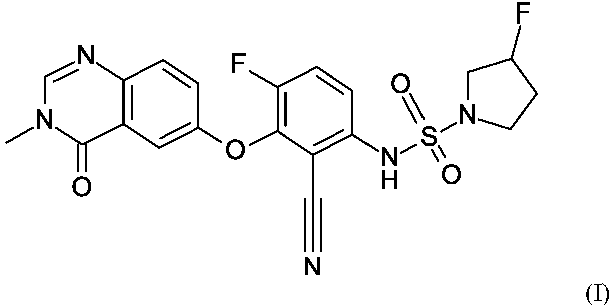 or a pharmaceutically acceptable salt or solvate thereof.

Any reference to a non-patentable method of treatment of the human or animal body by therapy involving a certain compound, combination, formulation or composition is to be understood as relating so said compound, combination, formulation or composition for use in said method of treatment.

Mutant BRAF is a targetable oncogenic driver and three BRAF inhibitors (BRAFi) up to date (Vemurafenib, Dabrafenib Encorafenib) reached the market showing efficacy in BRAFV600E-positive melanoma. However, rapid acquisition of drug resistance is almost universally observed and the duration of the therapeutic benefits for the targeted therapy remains limited.

Moreover, the developed first generation BRAF inhibitors revealed an unexpected and "paradoxical" ability to repress MAPK signalling in BRAF^{V600E}-driven tumors while the same inhibitors presented MAPK stimulatory activities in BRAF wild type (WT) models (N Engl J Med 2012; 366:271-273; and British Journal of Cancer volume 111, pages 640-645 (2014)).

Mechanistic studies on the RAF paradox then clarified that oncogenic BRAF^{V600E} phosphorylates MEK 1/2 in its monomeric cytosolic form while WT BRAF and RAF1 activation requires a complex step of events including cell membrane translocation and homo and/or heterodimerization promoted by activated RAS (KRAS, NRAS, HRAS) (Nature Reviews Cancer volume 14, pages 445-467 (2014)).

The binding of first generation BRAF inhibitors like Vemurafenib, Dabrafenib and Encorafenib to a WT BRAF or RAF1 protomer, quickly induces RAF homo and/or hetero dimerization and membrane association of the newly formed RAF dimer. In the dimeric conformation, one RAF protomer allosterically induces conformational changes of the second resulting in a kinase active status and, importantly, in a conformation unfavourable for the binding of the inhibitor. The dimer induced by drug treatment, as a result, promotes MEK phosphorylation by the catalysis operated by the unbound protomer with hyperactivation of the pathway.

Tumors almost inevitable evade from BRAFi treatment and the mechanism in the vast majority of cases involve the acquired ability of triggering RAF dimerization. This effect can be counteracted by MEK inhibitor (MEKi) combined treatment. These agents, however, present very poor therapeutic index which limits MEKi achievable doses in human. As a result, resistance to the combination therapy of a first generation BRAFi with a MEKi is still mediated by RAF paradoxical activation. The clinical benefit of those combination therapies remains thus limited.

Recently, it has been discovered that T cell dysfunction or anergy occurs concurrently with an induced and sustained expression of the inhibitory receptor, programmed death 1 polypeptide (PD-1). One of its ligands, PD-L1 is overexpressed in many cancers and is often associated with poor prognosis (Okazaki T et al., Intern. Immun. 2007 19(7):813) (Thompson RH et al., Cancer Res 2006, 66(7):3381). Interestingly, the majority of tumor infiltrating T lymphocytes predominantly express PD-1, in contrast to T lymphocytes in normal tissues and peripheral blood T lymphocytes indicating that up-regulation of PD-1 on tumor-reactive T cells can contribute to impaired antitumor immune responses (Blood 2009 1 14(8): 1537).

WO 2018/075447 A1 discloses a method for treating a solid malignant tumor (cancer) involving the use of a combination of a BRAF inhibitor and an immune checkpoint inhibitor, including a PD-1-antagonist, such as an anti-PD-1 antibody.

The present invention relates to a new combination of a BRAF inhibitor of formula (I) and a PD-1 axis binding antagonist for use in the treatment of cancer, in particular of melanoma, non-small cell lung cancer and leptomeningeal cancer. The compound of formula (I) is a BRAF inhibitor, which is showing neglectible paradoxical activation of the MAPK signaling pathway (paradox breaker) when compared to the first generation BRAF inhibitors that are on the market: Encorafenib, Dabrafenib and Vemurafenib (paradox inducers). In addition to this property, the compound of formula (I) also has very potent brain penetration properties, thus providing an urgently needed alternative therapy for the treatment of leptomeningial cancer or cancers which metastasized in the brain. The present invention discloses a new combination for use in cancer therapy with strong combined activity on BRAF associated tumours with the potential to overcome the rapidly acquired treatment resistance frequently observed in patients treated with first generation BRAF inhibitors. The combination as disclosed in the present invention for use in the treatment of cancer, presents unexpected combination activity that go far beyond the additive effects of BRAF inhibitor and PD-1 axis binding antagonist monotherapies.

### Brief description of Figures:

**Figure 1** reports the activity of either Compound Ia or the paradox inducer encorafenib alone or in combination with the MEKi cobimetinib on P-ERK levels in the cell model resistant to first generation BRAFi A375 BRAF^{V600E/}NRAS^{Q61K}.
**Figure 2** displays a schematic representation of the study design reported in Figures 3 and 4.

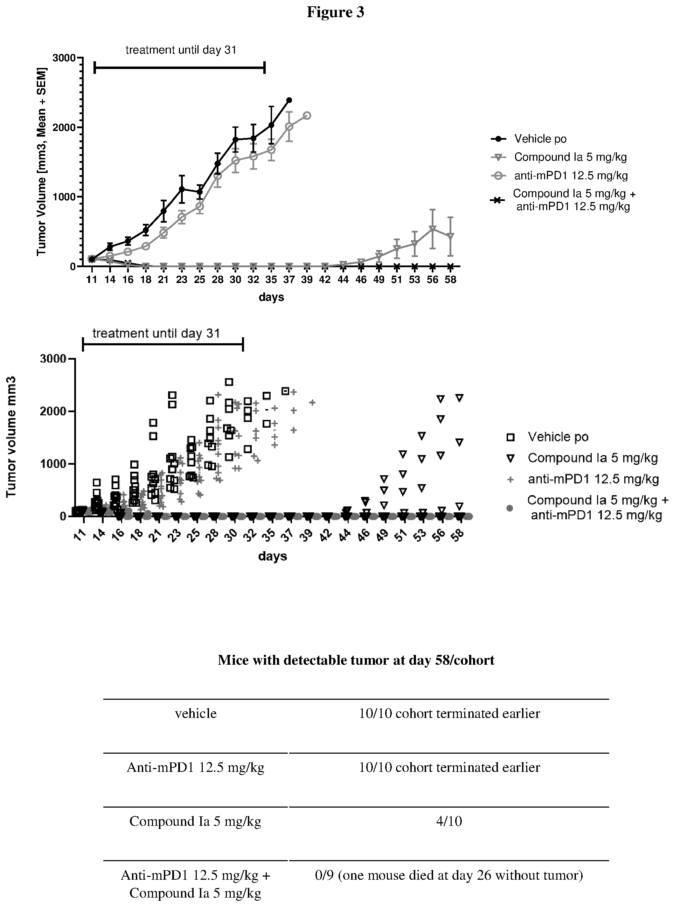

**Figure 3** reports in vivo antitumor activity of either Compound Ia at 5 mg/kg, anti mouse PD1 at 12.5 mg/kg and their combination on allograft tumors derived from the syngeneic model YUMM1.7. Mice were treated until day 31 and afterwards drug treatments were suspended and mice were monitored for evidences of tumor regrowth. The graph on the top expresses mean tumour growth [mm3] for the different cohorts. The graph on the middle expresses tumour growth of individual mice of the different cohorts. The table on the bottom summarizes the mice with detectable tumour at day 58 in the respective cohorts.

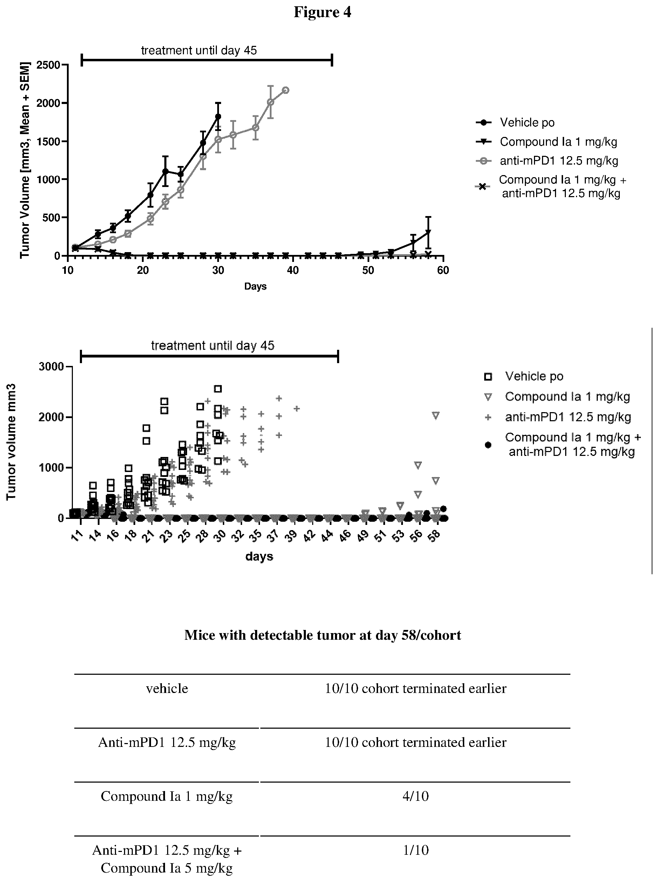

**Figure 4** reports in vivo antitumor activity of either Compound Ia at 1 mg/kg, anti mouse PD1 at 12.5 mg/kg and their combination on allograft tumors derived from the syngeneic model YUMM1.7. Mice were treated until day 45 and afterwards drug treatments were suspended and mice were monitored for evidences of tumor regrowth. The graph on the top expresses mean tumour growth [mm3] for the different cohorts. The graph on the middle expresses tumour growth of individual mice of the different cohorts. The table on the bottom summarizes the mice with detectable tumour at day 58 in the respective cohorts.
**Figure 5** displays a schematic diagram of the mice treatment for the immuno-phenotyping experiment reported in Figures 6 and 7.
**Figure 6** reports the total immune cell (top) and T-cell (bottom) infiltrates in tumors, as quantified through flow cytometry.Total immune cell infiltrate was expressed as percentage of mCD45 positive with respect to the alive cells at day 3 and day 6, and T-cell infiltrate expressed in mCD3 positive cells per mm2.
**Figure 7** reports the immunohistochemistry analysis of the mouse immune cell marker CD45 of tumors treated for 3 days with either Vehicle, Compound Ia at 5 mg/kg (daily), mPD1 at 12.5 mg/kg (single treatment) or a combination thereof.

The term "IC50" refers to the concentration of a particular compound required to inhibit 50% of a specific measured activity.

The term "inhibitor" denotes a compound which competes with, reduces or prevents the binding of a particular ligand to particular receptor, or which reduces or prevents the function of a particular protein. In particular, an inhibitor as used therein refers to compounds which target, decrease or inhibit activity of the respective target, particular inhibitors have an IC50 value below 1 µM, below 500 nM, below 200 nM, below 100 nM, below 50 nM, below 25 nM, below 10 nM, below 5 nM, 2 nM or below 1 nM.

The term "pharmaceutically acceptable salt" refers to those salts of the compound of formula (I) which retain the biological effectiveness and properties of the free bases or free acids, which are not biologically or otherwise undesirable. These salts can for instance be formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of a compound of formula (I) are the hydrochloride salts, methanesulfonic acid salts and citric acid salts.

The term "solvate" refers to non-covalent stoichiometric or nonstoichiometric combinations of solvent and solute. The term "hydrate" refers to non-covalent stoichiometric or nonstoichiometric combinations of water and solute. For example, compounds of formula (I) and pharmaceutically acceptable salts thereof, can exist in unsolvated as well as solvated forms with pharmaceutically acceptable solvents such as anisole, dichloromethane, toluene, 1,4-dioxane, water, and the like.

The compound of formula (I) contains one asymmetric center and can be present in the form of optically pure enantiomers or mixtures of enantiomers such as, for example, racemates.

According to the Cahn-Ingold-Prelog Convention the asymmetric carbon atom can be of the "R" or "S" configuration.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some aspects, the number of amino acid changes are 10 or less, 9 or less, 8 or less, 7 or less, 6 or less, 5 or less, 4 or less, 3 or less, or 2 or less. In some aspects, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (K_{D}). Affinity can be measured by common methods known in the art, including those described herein. Specific illustrative and exemplary methods for measuring binding affinity are described in the following.

An "affinity matured" antibody refers to an antibody with one or more alterations in one or more complementary determining regions (CDRs), compared to a parent antibody which does not possess such alterations, such alterations resulting in an improvement in the affinity of the antibody for antigen.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv, and scFab); single domain antibodies (dAbs); and multispecific antibodies formed from antibody fragments. For a review of certain antibody fragments, see Holliger and Hudson, Nature Biotechnology 23:1126-1136 (2005).

The term "linker" as used herein refers to a peptide linker and is preferably a peptide with an amino acid sequence with a length of at least 5 amino acids, preferably with a length of 5 to 100, more preferably of 10 to 50 amino acids. In one embodiment said peptide linker is (GₓS)ₙ or (GₓS)ₙGₘ with G = glycine, S = serine, and (x = 3, n= 3, 4, 5 or 6, and m= 0, 1, 2 or 3) or (x = 4,n= 2, 3, 4 or 5 and m= 0, 1, 2 or 3), preferably x = 4 and n= 2 or 3, more preferably with x = 4, n= 2. In one embodiment said peptide linker is (G₄S)₂.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ), based on the amino acid sequence of its constant domain. An immunoglobulin essentially consists of two Fab molecules and an Fc domain, linked via the immunoglobulin hinge region.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "antigen binding domain" refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more antibody variable domains (also called antibody variable regions). Preferably, an antigen binding domain comprises an antibody light chain variable region (VL) and an antibody heavy chain variable region (VH).

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. In certain aspects, the antibody is of the IgG₁ isotype. In certain aspects, the antibody is of the IgG₁ isotype with the P329G, L234A and L235A mutation to reduce Fc-region effector function. In other aspects, the antibody is of the IgG₂ isotype. In certain aspects, the antibody is of the IgG₄ isotype with the S228P mutation in the hinge region to improve stability of IgG₄ antibody. The heavy chain constant domains that correspond to the different classes of immunoglobulins are called α, δ, ε, γ, and µ, respectively. The light chain of an antibody may be assigned to one of two types, called kappa (κ) and lambda (λ), based on the amino acid sequence of its constant domain.

The terms "constant region derived from human origin" or "human constant region" as used in the current application denotes a constant heavy chain region of a human antibody of the subclass IgG1, IgG2, IgG3, or IgG4 and/or a constant light chain kappa or lambda region. Such constant regions are well known in the state of the art and e.g. described by Kabat, E.A., et al., Sequences of Proteins of Immunological Interest, 5th ed., Public Health Service, National Institutes of Health, Bethesda, MD (1991) (see also e.g. Johnson, G., and Wu, T.T., Nucleic Acids Res. 28 (2000) 214-218; Kabat, E.A., et al., Proc. Natl. Acad. Sci. USA 72 (1975) 2785-2788). Unless otherwise specified herein, numbering of amino acid residues in the constant region is according to the EU numbering system, also called the EU index of Kabat, as described in Kabat, E.A. et al., Sequences of Proteins of Immunological Interest, 5th ed., Public Health Service, National Institutes of Health, Bethesda, MD (1991), NIH Publication 91-3242.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g., B cell receptor); and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino- and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, G₃₂₉, P329G, or Pro329Gly.

An "effective amount" of an agent, e.g., a pharmaceutical composition, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one aspect, a human IgG heavy chain Fc region extends from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain. This may be the case where the final two C-terminal amino acids of the heavy chain are glycine (G446) and lysine (K447, EU numbering system). Therefore, the C-terminal lysine (Lys447), or the C-terminal glycine (Gly446) and lysine (Lys447), of the Fc region may or may not be present. Amino acid sequences of heavy chains including an Fc region are denoted herein without C-terminal glycine-lysine dipeptide if not indicated otherwise. In one aspect, a heavy chain including an Fc region as specified herein, comprised in an antibody according to the invention, comprises an additional C-terminal glycine-lysine dipeptide (G446 and K447, EU numbering system). In one aspect, a heavy chain including an Fc region as specified herein, comprised in an antibody according to the invention, comprises an additional C-terminal glycine residue (G446, numbering according to EU index). Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

"Framework" or "FR" refers to variable domain residues other than complementary determining regions (CDRs). The FR of a variable domain generally consists of four FR domains: FR1, FR2, FR3, and FR4. Accordingly, the CDR and FR sequences generally appear in the following sequence in VH (or VL): FR1-CDR-H1(CDR-L1)-FR2- CDR-H2(CDR-L2)-FR3-CDR-H3(CDR-L3)-FR4.

The terms "full length antibody", "intact antibody", and "whole antibody" are used herein interchangeably to refer to an antibody having a structure substantially similar to a native antibody structure or having heavy chains that contain an Fc region as defined herein.

The terms "host cell", "host cell line", and "host cell culture" are used interchangeably and refer to cells into which exogenous nucleic acid has been introduced, including the progeny of such cells. Host cells include "transformants" and "transformed cells", which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to *in vivo* somatic hypermutation. Thus, the amino acid sequences of the VH and VL regions of the recombinant antibodies are sequences that, while derived from and related to human germ line VH and VL sequences, may not naturally exist within the human antibody germ line repertoire *in vivo.*

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one aspect, for the VL, the subgroup is subgroup kappa I as in Kabat et al., *supra.* In one aspect, for the VH, the subgroup is subgroup III as in Kabat et al., *supra.*

A "humanized" antibody refers to a chimeric antibody comprising amino acid residues from non-human CDRs and amino acid residues from human FRs. In certain aspects, a humanized antibody will comprise substantially all of at least one, and typically two, variable domains, in which all or substantially all of the CDRs correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence and which determine antigen binding specificity, for example "complementarity determining regions" ("CDRs").

Generally, antibodies comprise six CDRs: three in the VH (CDR-H1, CDR-H2, CDR-H3), and three in the VL (CDR-L1, CDR-L2, CDR-L3). Exemplary CDRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991)); and
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)).

Unless otherwise indicated, the CDRs are determined according to Kabat et al., *supra.* One of skill in the art will understand that the CDR designations can also be determined according to Chothia, *supra,* McCallum, *supra,* or any other scientifically accepted nomenclature system.

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain aspects, the individual or subject is a human.

An "isolated" antibody is one which has been separated from a component of its natural environment. In some aspects, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC) methods. For a review of methods for assessment of antibody purity, see, e.g., Flatman et al., J. Chromatogr. B 848:79-87 (2007).

The term "nucleic acid molecule" or "polynucleotide" includes any compound and/or substance that comprises a polymer of nucleotides. Each nucleotide is composed of a base, specifically a purine- or pyrimidine base (i.e. cytosine (C), guanine (G), adenine (A), thymine (T) or uracil (U)), a sugar (i.e. deoxyribose or ribose), and a phosphate group. Often, the nucleic acid molecule is described by the sequence of bases, whereby said bases represent the primary structure (linear structure) of a nucleic acid molecule. The sequence of bases is typically represented from 5' to 3'. Herein, the term nucleic acid molecule encompasses deoxyribonucleic acid (DNA) including e.g., complementary DNA (cDNA) and genomic DNA, ribonucleic acid (RNA), in particular messenger RNA (mRNA), synthetic forms of DNA or RNA, and mixed polymers comprising two or more of these molecules. The nucleic acid molecule may be linear or circular. In addition, the term nucleic acid molecule includes both, sense and antisense strands, as well as single stranded and double stranded forms. Moreover, the herein described nucleic acid molecule can contain naturally occurring or non-naturally occurring nucleotides. Examples of non-naturally occurring nucleotides include modified nucleotide bases with derivatized sugars or phosphate backbone linkages or chemically modified residues. Nucleic acid molecules also encompass DNA and RNA molecules which are suitable as a vector for direct expression of an antibody of the invention *in vitro* and/or *in vivo,* e.g., in a host or patient. Such DNA (e.g., cDNA) or RNA (e.g., mRNA) vectors, can be unmodified or modified. For example, mRNA can be chemically modified to enhance the stability of the RNA vector and/or expression of the encoded molecule so that mRNA can be injected into a subject to generate the antibody *in vivo* (see e.g., Stadler ert al, Nature Medicine 2017, published online 12 June 2017, doi:10.1038/nm.4356 or EP 2 101 823 B1).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical composition.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable domain (VH), also called a variable heavy domain or a heavy chain variable region, followed by three constant heavy domains (CH1, CH2, and CH3). Similarly, from N- to C-terminus, each light chain has a variable domain (VL), also called a variable light domain or a light chain variable region, followed by a constant light (CL) domain.

A "blocking" antibody or an "antagonist" antibody is one that inhibits or reduces a biological activity of the antigen it binds. In some embodiments, blocking antibodies or antagonist antibodies substantially or completely inhibit the biological activity of the antigen. For example, the anti-PD-Ll antibodies of the invention block the signaling through PD- 1 so as to restore a functional response by T-cells (e.g., proliferation, cytokine production, target cell killing) from a dysfunctional state to antigen stimulation.

An "agonist" or activating antibody is one that enhances or initiates signaling by the antigen to which it binds. In some embodiments, agonist antibodies cause or activate signaling without the presence of the natural ligand.

The term "package insert" is used to refer to instructions customarily included in commercial packages of therapeutic products, that contain information about the indications, usage, dosage, administration, combination therapy, contraindications and/or warnings concerning the use of such therapeutic products.

"No substantial cross-reactivity" means that a molecule (*e.g*., an antibody) does not recognize or specifically bind an antigen different from the actual target antigen of the molecule (e.g. an antigen closely related to the target antigen), particularly when compared to that target antigen. For example, an antibody may bind less than about 10% to less than about 5% to an antigen different from the actual target antigen, or may bind said antigen different from the actual target antigen at an amount consisting of less than about 10%, 9%, 8% 7%, 6%, 5%, 4%, 3%, 2%, 1%, 0.5%, 0.2%, or 0.1%, preferably less than about 2%, 1%, or 0.5%, and most preferably less than about 0.2% or 0.1% antigen different from the actual target antigen.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity for the purposes of the alignment. Alignment for purposes of determining percent amino acid sequence identity can be achieved in various ways that are within the skill in the art, for instance, using publicly available computer software such as BLAST, BLAST-2, Clustal W, Megalign (DNASTAR) software or the FASTA program package. Those skilled in the art can determine appropriate parameters for aligning sequences, including any algorithms needed to achieve maximal alignment over the full length of the sequences being compared. Alternatively, the percent identity values can be generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087 and is described in WO 2001/007611.

Unless otherwise indicated, for purposes herein, percent amino acid sequence identity values are generated using the ggsearch program of the FASTA package version 36.3.8c or later with a BLOSUM50 comparison matrix. The FASTA program package was authored by W. R. Pearson and D. J. Lipman (1988), "Improved Tools for Biological Sequence Analysis", PNAS 85:2444-2448; W. R. Pearson (1996) "Effective protein sequence comparison" Meth. Enzymol. 266:227-258; and Pearson et. al. (1997) Genomics 46:24-36 and is publicly available from www.fasta.bioch.virginia.edu/fasta_www2/fasta_down.shtml or www. ebi.ac.uk/Tools/sss/fasta. Alternatively, a public server accessible at fasta.bioch.virginia.edu/fasta_www2/index.cgi can be used to compare the sequences, using the ggsearch (global protein:protein) program and default options (BLOSUM50; open: -10; ext: -2; Ktup = 2) to ensure a global, rather than local, alignment is performed. Percent amino acid identity is given in the output alignment header.

The term "pharmaceutical composition" or "pharmaceutical formulation" refers to a preparation which is in such form as to permit the biological activity of an active ingredient contained therein to be effective, and which contains no additional components which are unacceptably toxic to a subject to which the pharmaceutical composition would be administered.

A "pharmaceutically acceptable carrier" refers to an ingredient in a pharmaceutical composition or formulation, other than an active ingredient, which is nontoxic to a subject. A pharmaceutically acceptable carrier includes, but is not limited to, a buffer, excipient, stabilizer, or preservative.

The term "PD-1 axis binding antagonist" is a molecule that inhibits the interaction of a PD- 1 axis binding partner with either one or more of its binding partner, so as to remove T-cell dysfunction resulting from signaling on the PD-1 signaling axis - with a result being to restore or enhance T-cell function {e.g., proliferation, cytokine production, target cell killing). As used herein, a PD-1 axis binding antagonist includes a PD-1 binding antagonist, a PD-L1 binding antagonist and a PD-L2 binding antagonist.

The term "PD-1 binding antagonists" is a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to its binding partners. In a specific aspect, the PD-1 binding antagonist inhibits the binding of PD- 1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD- 1 with PD-L1 and/or PD-L2. In one embodiment, a PD-1 binding antagonist reduces the negative co-stimulatory signal mediated by or through cell surface proteins expressed on T lymphocytes mediated signaling through PD-1 so as render a dysfunctional T-cell less dysfunctional (e.g. , enhancing effector responses to antigen recognition). In some embodiments, the PD-1 binding antagonist is an anti-PD-1 antibody. In a specific aspect, a PD- 1 binding antagonist is MDX- 1106 described herein. In another specific aspect, a PD-1 binding antagonist is Merck 3745 described herein. The term "PD-L1 binding antagonists" is a molecule that decreases, blocks, inhibits, abrogates or interferes with signal transduction resulting from the interaction of PD-L1 with either one or more of its binding partners, such as PD-1 , B7-1 . In some embodiments, a PD-L1 binding antagonist is a molecule that inhibits the binding of PD-L 1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7- 1. In one embodiment, a PD-L1 binding antagonist reduces the negative co-stimulatory signal mediated by or through cell surface proteins expressed on T lymphocytes mediated signaling through PD- L 1 so as to render a dysfunctional T-cell less dysfunctional (e.g. , enhancing effector responses to antigen recognition). In some embodiments, a PD-L1 binding antagonist is an anti-PD-L 1 antibody. In a specific aspect, an anti-PD-L1 antibody is YW243.55.S70 described herein. In another specific aspect, an anti-PD-L1 antibody is MDX- 1 105 described herein. In still another specific aspect, an anti-PD-L1 antibody is MPDL3280A described herein.

The term "PD-L2 binding antagonists" is a molecule that decreases, blocks, inhibits, abrogates or interferes with signal transduction resulting from the interaction of PD-L2 with either one or more of its binding partners, such as PD- 1. In some embodiments, a PD-L2 binding antagonist is a molecule that inhibits the binding of PD-L2 to its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD- L2 with either one or more of its binding partners, such as PD-1. In one embodiment, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g. , enhancing effector responses to antigen recognition). In some embodiments, a PD-L2 binding antagonist is an immunoadhesin.

A "PD-1 oligopeptide", "PD-L1 oligopeptide" or "PD-L2 oligopeptide" is an oligopeptide that binds, preferably specifically, to a PD-1 , PD-L1 or PD-L2 negative costimulatory polypeptide, respectively, including a receptor, ligand or signaling component, respectively, as described herein. Such oligopeptides may be chemically synthesized using known oligopeptide synthesis methodology or may be prepared and purified using recombinant technology. Such oligopeptides are usually at least about 5 amino acids in length, alternatively at least about 6, 7, 8, 9, 10, 1 1 , 12, 13, 14, 15, 16, 17, 18, 19, 20, 21 , 22, 23, 24, 25, 26, 27, 28, 29, 30, 31 , 32, 33, 34, 35, 36, 37, 38, 39, 40, 41 , 42, 43, 44, 45, 46, 47, 48, 49, 50, 51 , 52, 53, 54, 55, 56, 57, 58, 59, 60, 61 , 62, 63, 64, 65, 66, 67, 68, 69, 70, 71 , 72, 73, 74, 75, 76, 77, 78, 79, 80, 81 , 82, 83, 84, 85, 86, 87, 88, 89, 90, 91 , 92, 93, 94, 95, 96, 97, 98, 99, or 100 amino acids in length or more. Such oligopeptides may be identified using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871 , 4,833,092, 5,223,409, 5,403,484, 5,571 ,689, 5,663, 143; PCT Publication Nos. WO 84/03506 and WO84/03564; Geysen et al., Proc. Natl. Acad. Sci. U.S.A., 81:3998-4002 (1984); Geysen et al, Proc. Natl. Acad. Sci. U. S.A., 82: 178-182 (1985); Geysen et al, in Synthetic Peptides as Antigens, 130-149 (1986); Geysen et al., J. Immunol. Metk, 102:259-274 (1987); Schoofs et al., J. Immunol., 140:61 1 -616 (1988), Cwirla, S. E. et al. Proc. Natl. Acad. Sci. USA, 87:6378 (1990); Lowman, H.B. et al. Biochemistry, 30: 10832 ( 1991 ); Clackson, T. et al. Nature, 352: 624 (1991); Marks, J. D. et al., J. Mol. Biol., 222:581 (1991); Kang, A.S. et al. Proc. Natl. Acad. Sci. USA, 88:8363 (1991), and Smith, G. P., Current Opin. Biotechnol, 2:668 (1991).

The term "anergy" refers to the state of unresponsiveness to antigen stimulation resulting from incomplete or insufficient signals delivered through the T-cell receptor (e.g. increase in intracellular Ca⁺² in the absence of ras-activation). T cell anergy can also result upon stimulation with antigen in the absence of co-stimulation, resulting in the cell becoming refractory to subsequent activation by the antigen even in the context of costimulation. The unresponsive state can often be overriden by the presence of Interleukin-2. Anergic T-cells do not undergo clonal expansion and/or acquire effector functions.

The term "exhaustion" refers to T cell exhaustion as a state of T cell dysfunction that arises from sustained TCR signaling that occurs during many chronic infections and cancer. It is distinguished from anergy in that it arises not through incomplete or deficient signaling, but from sustained signaling. It is defined by poor effector function, sustained expression of inhibitory receptors and a transcriptional state distinct from that of functional effector or memory T cells. Exhaustion prevents optimal control of infection and tumors. Exhaustion can result from both extrinsic negative regulatory pathways (e.g., immunoregulatory cytokines) as well as cell intrinsic negative regulatory (costimulatory) pathways (PD-1 , B7-H3, B7-H4, etc.).

"Enhancing T-cell function" means to induce, cause or stimulate a T-cell to have a sustained or amplified biological function, or renew or reactivate exhausted or inactive T-cells. Examples of enhancing T-cell function include: increased secretion of γ-interferon from CD8⁺ T-cells, increased proliferation, increased antigen responsiveness (e.g. , viral, pathogen, or tumor clearance) relative to such levels before the intervention. In one embodiment, the level of . enhancement is as least 50%, alternatively 60%, 70%, 80%, 90%, 100%, 1 20%, 150%, 200%. The manner of measuring this enhancement is known to one of ordinary skill in the art.

"Tumor immunity" refers to the process in which tumors evade immune recognition and clearance. Thus, as a therapeutic concept, tumor immunity is "treated" when such evasion is attenuated, and the tumors are recognized and attacked by the immune system. Examples of tumor recognition include tumor binding, tumor shrinkage and tumor clearance.

"Immunogenecity" refers to the ability of a particular substance to provoke an immune response. Tumors are immunogenic and enhancing tumor immunogenicity aids in the clearance of the tumor cells by the immune response. Examples of enhancing tumor immunogenicity include treatment with anti-PDL antibodies and a ME inhibitor.

"Sustained response" refers to the sustained effect on reducing tumor growth after cessation of a treatment. For example, the tumor size may remain to be the same or smaller as compared to the size at the beginning of the administration phase. In some embodiments, the sustained response has a duration at least the same as the treatment duration, at least 1 .5X, 2. OX, 2.5X, or 3. OX length of the treatment duration.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some aspects, antibodies of the invention are used to delay development of a disease or to slow the progression of a disease.

The term "cancer" as used herein refers to proliferative diseases, such as the cancer is colorectal cancer, sarcoma, head and neck cancer, squamous cell carcinoma, breast cancer, pancreatic cancer, gastric cancer, non-small-cell lung carcinoma, small-cell lung cancer and mesothelioma, including refractory versions of any of the above cancers, or a combination of one or more of the above cancers. In one embodiment, the cancer is colorectal cancer and optionally the chemotherapeutic agent is Irinotecan. In embodiments in which the cancer is sarcoma, optionally the sarcoma is chondrosarcoma, leiomyosarcoma, gastrointestinal stromal tumours, fibrosarcoma, osteosarcoma. liposarcoma or maligant fibrous histiocytoma.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three complementary determining regions (CDRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors". As used herein, the term "antigen binding molecule" refers in its broadest sense to a molecule that specifically binds an antigenic determinant. Examples of antigen binding molecules are immunoglobulins and derivatives, e.g. fragments, thereof.

The term "antigen-binding site of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

Antibody specificity refers to selective recognition of the antibody for a particular epitope of an antigen. Natural antibodies, for example, are monospecific. "Bispecific antibodies" according to the invention are antibodies which have two different antigen-binding specificities. Antibodies of the present invention are specific for two different antigens, i.e. DR5 as first antigen and FAP as second antigen.

The term "monospecific" antibody as used herein denotes an antibody that has one or more binding sites each of which bind to the same epitope of the same antigen.

The term "bispecific" means that the antigen binding molecule is able to specifically bind to at least two distinct antigenic determinants. Typically, a bispecific antigen binding molecule comprises at least two antigen binding sites, each of which is specific for a different antigenic determinant. In certain embodiments the bispecific antigen binding molecule is capable of simultaneously binding two antigenic determinants, particularly two antigenic determinants expressed on two distinct cells.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising at least one antigen binding site that binds to FAP or DR5 as well as another, different antigen (see, US 2008/0069820, for example).

The term "valent" as used within the current application denotes the presence of a specified number of binding sites in an antibody molecule. As such, the terms "bivalent", "tetravalent", and "hexavalent" denote the presence of two binding sites, four binding sites, and six binding sites, respectively, in an antibody molecule. The bispecific antibodies according to the invention are at least "bivalent" and may be "trivalent" or "multivalent" (e.g. "tetravalent" or "hexavalent").

Antibodies of the present invention have two or more binding sites and are bispecific. That is, the antibodies may be bispecific even in cases where there are more than two binding sites (i.e. that the antibody is trivalent or multivalent). Bispecific antibodies of the invention include, for example, multivalent single chain antibodies, diabodies and triabodies, as well as antibodies having the constant domain structure of full length antibodies to which further antigen-binding sites (e.g., single chain Fv, a VH domain and/or a VL domain, Fab, or (Fab)2) are linked via one or more peptide-linkers. The antibodies can be full length from a single species, or be chimerized or humanized.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "amino acid" as used within this application denotes the group of naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

As used herein, the expressions "cell", "cell line", and "cell culture" are used interchangeably and all such designations include progeny. Thus, the words "transfectants" and "transfected cells" include the primary subject cell and cultures derived there from without regard for the number of transfers. It is also understood that all progeny may not be precisely identical in DNA content, due to deliberate or inadvertent mutations. Variant progeny that have the same function or biological activity as screened for in the originally transformed cell are included.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

As used herein, the term "binding" or "specifically binding" refers to the binding of the antibody to an epitope of the antigen in an in-vitro assay, preferably in a surface plasmon resonance assay (SPR, BIAcore, GE-Healthcare Uppsala, Sweden). The affinity of the binding is defined by the terms ka (rate constant for the association of the antibody from the antibody/antigen complex), kD (dissociation constant), and KD (kD/ka). Binding or specifically binding means a binding affinity (KD) of 10⁻⁸ mol/l or less, preferably 10⁻⁹ M to 10⁻¹³ mol/l.

Binding of the antibody to the death receptor can be investigated by a BIAcore assay (GE-Healthcare Uppsala, Sweden). The affinity of the binding is defined by the terms ka (rate constant for the association of the antibody from the antibody/antigen complex), kD (dissociation constant), and KD (kD/ka)

"Reduced binding", for example reduced binding to an Fc receptor, refers to a decrease in affinity for the respective interaction, as measured for example by SPR. For clarity the term includes also reduction of the affinity to zero (or below the detection limit of the analytic method), i.e. complete abolishment of the interaction. Conversely, "increased binding" refers to an increase in binding affinity for the respective interaction.

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. Suitable assays to measure T cell activation are known in the art described herein.

A "target cell antigen" as used herein refers to an antigenic determinant presented on the surface of a target cell, for example a cell in a tumor such as a cancer cell or a cell of the tumor stroma. In particular "target cell antigen" refers to Folate Receptor 1.

As used herein, the terms "first" and "second" with respect to antigen binding moieties etc., are used for convenience of distinguishing when there is more than one of each type of moiety.

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein, e.g., PD-1 and PD-L1, can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

As used herein, the terms "engineer, engineered, engineering," particularly with the prefix "glyco-," as well as the term "glycosylation engineering" are considered to include any manipulation of the glycosylation pattern of a naturally occurring or recombinant polypeptide or fragment thereof. Glycosylation engineering includes metabolic engineering of the glycosylation machinery of a cell, including genetic manipulations of the oligosaccharide synthesis pathways to achieve altered glycosylation of glycoproteins expressed in cells. Furthermore, glycosylation engineering includes the effects of mutations and cell environment on glycosylation. In one embodiment, the glycosylation engineering is an alteration in glycosyltransferase activity. In a particular embodiment, the engineering results in altered glucosaminyltransferase activity and/or fucosyltransferase activity.

The combination therapies in accordance with the invention have a synergistic effect. A "synergistic effect" of two compounds is one in which the effect of the combination of the two agents is greater than the sum of their individual effects and is statistically different from the controls and the single drugs. In another embodiment, the combination therapies disclosed herein have an additive effect. An "additive effect" of two compounds is one in which the effect of the combination of the two agents is the sum of their individual effects and is statistically different from either the controls and/or the single drugs.

In one aspect, the present invention provides a BRAF inhibitor and a PD-1 axis binding antagonist for use in the treatment of cancer, wherein the BRAF inhibitor is a compound of formula (I) 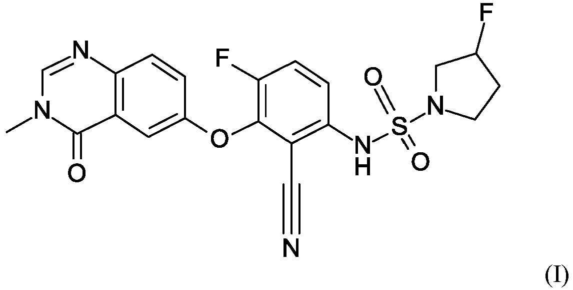 or a pharmaceutically acceptable salt or solvate thereof.

In some embodiments of the present invention the compound of formula (I) is a compound according to formula (Ia)

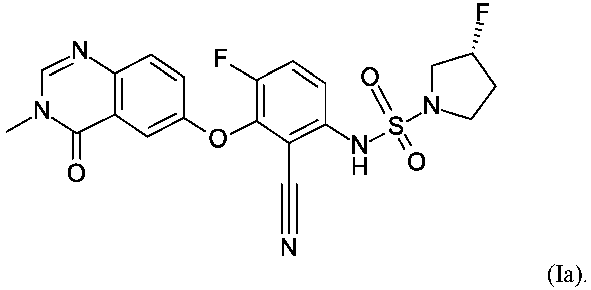

In some embodiments of the present invention the compound of formula (I) is a compound according to formula (Ib)

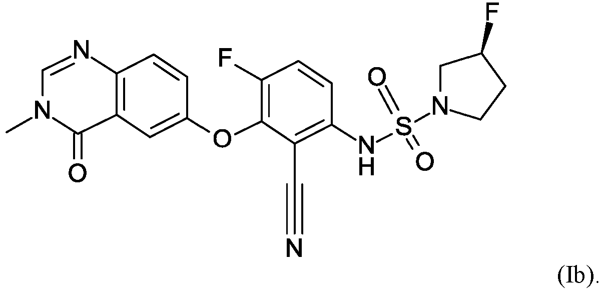

Non-limiting examples of PD-1 axis binding antagonist for the use according to the invention include cemiplimab (Libtayo^{®}), pembrolizumab (Keytruda^{®}), nivolumab (Opdivo^{®}), RN888 (PF-06801591), atezolizumab (Tecentriq^{®}), avelumab (Bavencio^{®}) and durvalumab (ImfinziTM).

In some embodiments of the present invention the PD-1 axis binding antagonist is a PD-1 binding antagonist selected from cemiplimab (Libtayo^{®}), pembrolizumab (Keytruda^{®}), nivolumab (Opdivo^{®}) and RN888 (PF-06801591). In some embodiments of the present invention the PD-1 axis binding antagonist is a PD-L1 binding antagonist selected from atezolizumab (Tecentriq^{®}), avelumab (Bavencio^{®}) and durvalumab (ImfinziTM).

### Assay procedures

### Materials

DMEM no-phenol red medium supplemented with L-glutamine was purchased from (Thermo Fisher Scientific). Fetal bovine serum (FBS) was purchased from VWR. Advanced ERK phospho-T202 /Y204 kit - 10,000 tests was purchased from Cisbio cat# 64AERPEH. A375 were originally obtained from ATCC and banked by the Roche repository. 384-well microplates were purchased from Greiner Bio-One, 384-well, (With Lid, HiBase, Low volume cat 784-080).

### HTRF assay for P-ERK determination in A375 cells or HCT116 cells

A375 is a cellular cancer model expressing V600E mutated BRAF and HCT116 a cellular cancer model expressing WT BRAF. First generation BRAF inhibitors such as e.g. dabrafenib induce a paradox effect on tumour cells in that they inhibit the growth of V600E mutated BRAF cells (such as e.g. A375), while they activate growth in WT BRAF cells (such as e.g. HCT 116). ERK 1,2 phosphorylation (terminal member of the phosphorylation cascade of the MAPK pathway) is hereafter reported as main readout for the activation status of the MAPK pathway. Prior to the assay, A375 and HCT116 cell lines are maintained in DMEM no-phenol red medium supplemented with 10% fetal bovine serum (FBS). Following compound treatment, P-ERK levels are determined by measuring FRET fluorescence signal induced by selective binding of 2 antibodies provided in the mentioned kit (Cisbio cat# 64AERPEH) on ERK protein when phosphorylated at Thr202/Tyr204. Briefly, 8000 cells/well in 12 µl media/well are plated in the 384-well plate and left overnight in the incubator (at 37 °C with 5% CO2-humidified atmosphere), the following day the plate is treated in duplicate with test compounds, dabrafenib and PLX8394 (the latter two as controls) at the following final drug concentrations: 10µM-3µM-1µM-0.3µM-0.1µM-0.03µM-0,01µM-0.003µM-0.001µM, all wells are subjected to DMSO normalization and drug incubation occurs for 1 hour. Then, 4µl of a 4X lysis buffer supplied with the kit are added to the wells, the plate is then centrifuged for 30 second (300 rcf) and incubated on a plate shaker for 1h at RT.

At the end of the incubation 4µL/well of advanced P-ERK antibody solution (prepared according to manufacturer's instruction) followed by 4µL/well of criptate P-ERK antibody solution (prepared according to manufacturer's instruction) (Cisbio cat# 64AERPEH) are added to test wells.

In order to allow proper data normalization control wells without drug treatment reported are always included in each plate (according to manufacturer's instruction):
p-ERK HTRF well content of controls and experimental (µl):

| neg ctrl | pos ctrl | neut ctrl | cpd | blank | |
|---|---|---|---|---|---|
| - | - | 12 | 12 | 12 | Cells |
| 12 | - | - | - | - | Media |
| - | - | - | <0.05 | - | Cpd |
| - | 16 | - | - | - | control lysate (ready-to-use) |
| 4 | - | 4 | 4 | 4 | 4x lysis buffer |
| 4 | 4 | 4 | 4 | - | Advanced p-ERK antibody solution |
| - | - | - | - | 4 | Advanced p-ERK1/2 Cryptate antibody solut. |
| 20 | 20 | 20 | 20 | 20 | Total volume in Well |

The plate is then centrifuged at 300 rcf for 30 second, sealed to prevent evaporation and incubated overnight in the dark at room temperature.

The plate is then analyzed and fluorescence emission value collected through a Pherastast FSX (BMG Labtech) apparatus at 665 and 620 nM.

The obtained fluorescence values are processed according to the formula Ratio=Signal(620nm)/Signal(625nm)*10000 then the average of the ratio on the blank is subtracted to all values.

Data are normalized in the case of A375 cells (BRAF inhibition) considering the average of the ratio (blank subtracted) derived by DMSO only treated cells as 100% and by considering the average of the ratio (blank subtracted) derived by 10µM dabrafenib treated cells as 0%. Mean of the normalized points are fitted with sigmoidal curve and IC50 determined. The results are shown in Table 1.

Data are normalized in the case of HCT116 cells (BRAF activation,) considering the average of the ratio (blank subtracted) derived by DMSO only treated cells as 0% and by considering the average of the ratio (blank subtracted) derived by dabrafenib treated cells at the concentration which provides the highest signal as 100%. Individual points are fitted with either sigmoidal or bell shape curves, and the percentage of activation compared to maximum dabrafenib-mediated activation is determined. The EC50 is the concentration at which activation equal to 50% of the maximum achieved by dabrafenib is obtained. The results are shown in Table 2.

In case the activation does not reach 50% of the maximum achieved by dabrafenib, then the EC50 calculation is not applicable.

The Percentage of Maximum paradox inducing effect from dabrafenib is determined by evaluating the percentage at which the test compound induce its maximum P-ERK signal as percentage of the highest signal produced by dabrafenib within the dose range tested.
WO2012/118492 discloses references compounds AR-25 as example 25, AR-30 as example 30 and AR-31 as example 31.

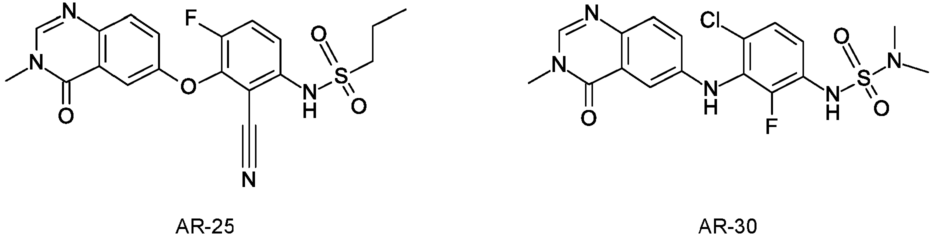

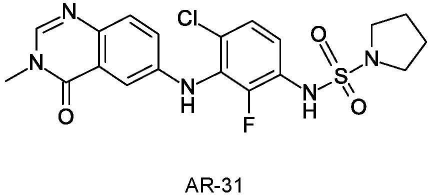

**Table 1: Ex. 1 ((3R)-N-[2-cyano-4-fluoro-3-(3-methyl-4-oxo-quinazolin-6-yl)oxy-phenyl]-3-fluoro-pyrrolidine-1-sulfonamide) has high affinity for RAF kinases and high selectivity over C-terminal Src kinase (CSK) and lymphocyte-specific tyrosine protein kinase (LCK), when compared to AR-30 and AR-31.**

| **Ex.** | **Kd (µM)** | | | | |
|---|---|---|---|---|---|
| | **BRAF** | **BRAF V600E** | **CRAF** | **CSK** | **LCK** |
| **1** | 0.0006 | 0.0012 | 0.0017 | 23.3 | 40 |
| **AR-25** | 0.0001 | 0.0002 | 0.0003 | >40 | >40 |
| **AR-30** | 0.1740 | 0.5040 | 0.8220 | 8.007 | 10.352 |
| **AR-31** | 0.0459 | 0.1190 | 0.1903 | 1.208 | 11.975 |

**Table 2: Ex. 1 ((3R)-N-[2-cyano-4-fluoro-3-(3-methyl-4-oxo-quinazolin-6-yl)oxy-phenyl]-3-fluoro-pyrrolidine-1-sulfonamide) is breaking the paradoxical RAF activation in HCT-116 cancer cells expressing WT BRAF. When compared with dabrafenib or with AR-25 the maximum paradox inducing effect is reduced to less than 50%.**

| **Ex.** | **pERK IC₅₀ (nM)** | **p-ERK EC50 (nM)** | **Percentage of Maximum paradox inducing effect from dabrafenib** |
|---|---|---|---|
| | | **conc. (nM) at which the compound induces P-ERK activation of 50% of that induced by dabrafenib (Positive control paradox inducer)** | |
| | **A375** | **HCT-116** | |
| **1** | 6.9 | not applicable | 43.65% |
| **AR-25** | 1.1 | 9.6 | 103% |
| **AR-30** | 406 | >1000 | 59% |
| **AR-31** | 311 | >1000 | 51.2% |

The preparation of the compound of formula (I) of the present invention may be carried out in sequential or convergent synthetic routes. Syntheses of the invention are shown in the following general scheme. The skills required for carrying out the reactions and purifications of the resulting products are known to those skilled in the art.

In more detail, the compound of formula (I) can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the experimental procedures below, or by methods known in the art.

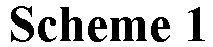

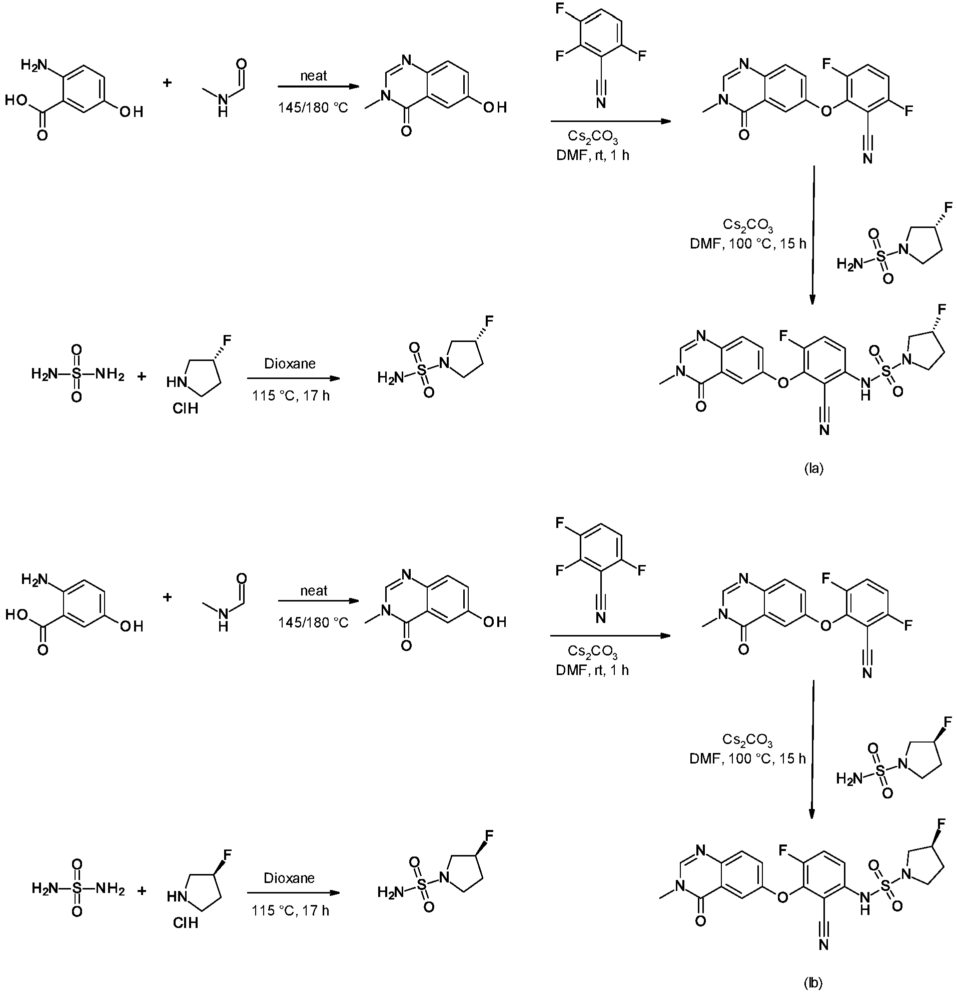

It will be appreciated that the compound of formula (I) in this invention may be derivatised at functional groups to provide derivatives which are capable of conversion back to the parent compound *in vivo.*

### Experimental Procedures

### (3R)-N-[2-cyano-4-fluoro-3-(3-methyl-4-oxo-quinazolin-6-yl)oxy-phenyl]-3-fluoro-pyrrolidine-1-sulfonamide (compound of formula (Ia)) and (3S)-N-[2-cyano-4-fluoro-3-(3-methyl-4-oxo-quinazolin-6-yl)oxy-phenyl]-3-fluoro-pyrrolidine-1-sulfonamide (compound of formula (Ib))

### 6-hydroxy-3-methyl-quinazolin-4-one

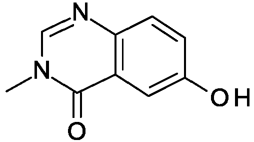

2-Amino-5-hydroxybenzoic acid (10 g, 65.3 mmol, Eq: 1.0) and N-methylformamide (30 g, 29.9 mL, 503 mmol, Eq: 7.7) were heated at 145 °C for 21 h 45 min, then cooled to rt. The reaction mixture was diluted with 50 mL H₂O and stirred at rt for 20 min. The resulting precipitate was collected by filtration. The light brown solid was washed 3 × with 20 mL water. The solid was taken up in toluene and evaporated to dryness (3 ×). The solid was dried in vacuo at 40 °C overnight under high vacuum to give the title compound as a light brown solid (10.3 g, 89% yield). MS (ESI) *m*/*z:* 177.1 [M+H]⁺.

### 3,6-difluoro-2-(3-methyl-4-oxo-quinazolin-6-yl)oxy-benzonitrile

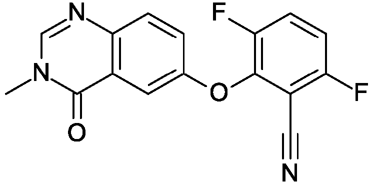

Cesium carbonate (3.22 g, 9.79 mmol, Eq: 1.15) was added at rt to a solution of 6-hydroxy-3-methylquinazolin-4-one (1500 mg, 8.51 mmol, Eq: 1.0) in N,N-dimethylformamide (35 mL). The mixture was stirred for 30 min at rt then 2,3,6-trifluorobenzonitrile (1.47 g, 1.08 ml, 9.37 mmol, Eq: 1.1) was added. After 1 h, the reaction was cooled on ice and diluted with water (120 mL). The resultant solid was collected by filtration, washed with iced water (100 mL) and heptane (100 mL) and suction-dried. The solid was taken up in toluene and evaporated to dryness (3 ×) then dried overnight in vacuo to give the title compound as a light brown solid (2.58 g, 97% yield). MS (ESI) *m*/*z:* 314.1 [M+H]+.

### (3R)-3-fluoropyrrolidine-1-sulfonamide

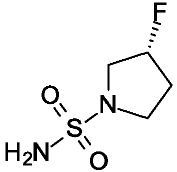

(R)-3-Fluoropyrrolidine hydrochloride (1.8 g, 14.3 mmol, Eq: 1.2) was added to a solution of sulfuric diamide (1.148 g, 11.9 mmol, Eq: 1.0) and triethylamine (2.42 g, 3.33 mL, 23.9 mmol, Eq: 2) in dioxane (10 mL). The reaction was stirred in a sealed tube at 115 °C for 15.5 h then cooled to rt and concentrated in vacuo. The residue was diluted with DCM, evaporated with silica gel to dryness and transferred to a column. Purification by flash chromatography (40 g silica, 80% EtOAc) gave the title compound as a white crystalline solid (1.82 g, 91% yield). MS (ESI) *m*/*z:* 169.1 [M+H]⁺.

### (3S)-3-fluoropyrrolidine-I-sulfonamide

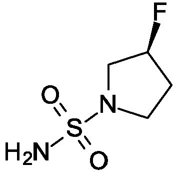

Triethylamine (304 mg, 419 µl, 3.01 mmol, Eq: 2.0) was added to a suspension of sulfuric diamide (146 mg, 1.5 mmol, Eq: 1.0) and (S)-3-fluoropyrrolidine hydrochloride (234 mg, 1.8 mmol, Eq: 1.2) in dioxane (1.3 ml). The reaction was stirred in a sealed tube at 115°C for 16 h 35 min, then concentrated in vacuo. The residue was diluted with MeOH and evaporated with silica gel to dryness and transferred to a column. Purification by flash chromatography (40 g silica, 0-8% MeOH/DCM) gave the title compound as a light yellow solid (193 mg, 75% yield). MS (ESI) *m*/*z:* 169.1 [M+H]⁺.

### (3R)-N-[2-cyano-4-fluoro-3-(3-methyl-4-oxo-quinazolin-6-yl)oxy-phenyl]-3-fluoro-pyrrolidine-1-sulfonamide (compound of formula (Ia))

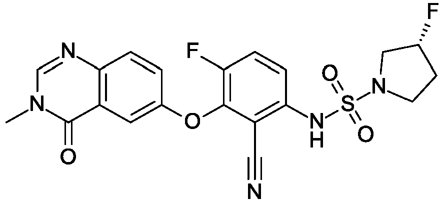

(*R*)-3-Fluoropyrrolidine-1-sulfonamide (1.26 g, 7.51 mmol, Eq: 2.1) and cesium carbonate (2.56 g, 7.87 mmol, Eq: 2.2) were suspended in dry DMF (10.2 ml) under an argon atmosphere. The reaction was stirred at 50 °C for 30 min. The reaction mixture was cooled to rt and a solution of 3,6-difluoro-2-((3-methyl-4-oxo-3,4-dihydroquinazolin-6-yl)oxy)benzonitrile (1.12 g, 3.58 mmol, Eq: 1.0) in DMF (25.5 ml) was added. The reaction mixture was stirred at 100 °C for 15 h, then concentrated in vacuo. The residue was taken up in sat. aq. NH₄Cl (100 mL) and EtOAc (100 mL). The phases were separated, and the aqueous layer was extracted further with 2 x 100 mL EtOAc. The combined organic layers were washed with water (200 mL) and brine (200 mL), dried (Na₂SO₄), filtered and concentrated in vacuo. The water layer was back-extracted with EtOAc (3 x 100 mL). The combined organic extracts were washed with brine (200 mL), dried (Na₂SO₄), filtered and concentrated in vacuo. The residue was diluted with DCM and MeOH, and concentrated onto silica. Purification by flash chromatography (120 g, 0.5-2% MeOH/DCM) gave an off-white solid which was triturated with 1:1 heptane/DCM (20 mL) with sonication, then dried in vacuo to give the title compound as a colourless solid (1.087 g, 66% yield). MS (ESI) *m*/*z:* 426.2 [M+H]⁺. Chiral SFC: RT = 4.594 min [Chiralpak IC column, 4.6 x 250 mm, 5µm particle size (Daicel); gradient of 20 - 40% MeOH containing 0.2% NHEt₂ over 8 min; flow: 2.5 mL/min; 140 bar backpressure].

### (3S)-N-[2-cyano-4-fluoro-3-(3-methyl-4-oxo-quinazolin-6-yl)oxy-phenyl]-3-fluoro-pyrrolidine-1-sulfonamide (compound of formula (Ib))

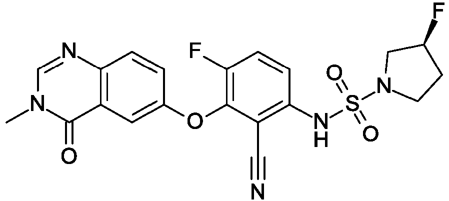

(*S*)-3-Fluoropyrrolidine-1-sulfonamide (181 mg, 1.08 mmol, Eq: 2.1) was dissolved in DMF (1.6 ml). At rt cesium carbonate (368 mg, 1.13 mmol, Eq: 2.2) was added and the reaction mixture was stirred at 50 °C for 30 min. The reaction mixture was cooled to rt and a solution of 3,6-difluoro-2-((3-methyl-4-oxo-3,4-dihydroquinazolin-6-yl)oxy)benzonitrile (160.8 mg, 513 µmol, Eq: 1.0) in DMF (4 ml) was added. The reaction mixture was stirred at 105 °C for 2 h 50 min then concentrated in vacuo. The residue was taken up in DCM and washed with sat. aq. NH₄Cl. The aq. layer was back-extracted twice with DCM. The combined organic layers were dried over Na₂SO₄, filtrated and evaporated. The residue (brown oil) was diluted with DCM and transferred to a column. Purification by flash chromatography (80 g, 0-100% EtOAc in DCM) gave a solid which was further purified by SFC to give the title compound as a light yellow solid (119 mg, 50% yield). MS (ESI) *m*/*z:* 426.2 [M+H]⁺. Chiral SFC: RT = 4.411 min [Chiralpak IC column, 4.6 x 250 mm, 5µm particle size (Daicel); gradient of 20 - 40% MeOH containing 0.2% NHEt₂ over 8 min; flow: 2.5 mL/min; 140 bar backpressure].

The invention relates in particular to:
A combination of a BRAF inhibitor and a PD-1 axis binding antagonist wherein the BRAF inhibitor is a compound of formula (I) 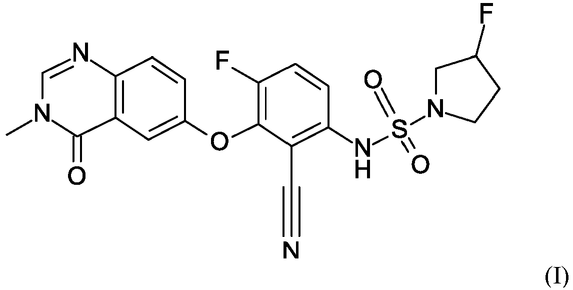 or a pharmaceutically acceptable salt or solvate thereof;
A combination of a BRAF inhibitor and a PD-1 axis binding antagonist as described herein, wherein the compound of formula (I) is (3R)-N-[2-cyano-4-fluoro-3-(3-methyl-4-oxo-quinazolin-6-yl)oxy-phenyl]-3-fluoro-pyrrolidine-1-sulfonamide;
A combination of a BRAF inhibitor and a PD-1 axis binding antagonist according to the invention, wherein the PD-1 axis binding antagonist is selected from the group consisting of a PD-1 binding antagonist, a PD-Ll binding antagonist and a PD-L2 binding antagonist;
A combination of a BRAF inhibitor and a PD-1 axis binding antagonist according to the invention, wherein the PD-1 axis binding antagonist is selected from cemiplimab (Libtayo^{®}), pembrolizumab (Keytruda^{®}), nivolumab (Opdivo^{®}), RN888 (PF-06801591), atezolizumab (Tecentriq^{®}), avelumab (Bavencio^{®}) and durvalumab (ImfinziTM);
A combination of a BRAF inhibitor and PD-1 axis binding antagonist according to the invention, wherein the PD-1 axis binding antagonist is a PD-1 binding antagonist selected from cemiplimab (Libtayo^{®}), pembrolizumab (Keytruda^{®}), nivolumab (Opdivo^{®}) and RN888 (PF-06801591);
A combination of a BRAF inhibitor and PD-1 axis binding antagonist according to the invention, wherein the PD-1 axis binding antagonist is a PD-L1 inhibitor selected from atezolizumab (Tecentriq^{®}), avelumab (Bavencio^{®}) and durvalumab (ImfinziTM);
A combination of a BRAF inhibitor and PD-1 axis binding antagonist according to the invention, wherein the PD-1 axis binding antagonist is atezolizumab (Tecentriq^{®});
A combination of a BRAF inhibitor and a PD-1 axis binding antagonist according to the invention herein, for use as a medicament;
A combination of a BRAF inhibitor and a PD-1 axis binding antagonist according to the invention, for use in the therapeutic and/or prophylactic treatment of cancer, in particular melanoma, non-small cell lung cancer or leptomeningeal cancer;
A pharmaceutical composition comprising a combination of a BRAF inhibitor and a PD-1 axis binding antagonist according to the invention, and one or more pharmaceutically acceptable excipients;
A combination or a pharmaceutical composition according to the invention, wherein the BRAF inhibitor is administere orally and the PD-1 axis binding antagonist is administered by injection, such as intravenous or subcutaneous injection;
A combination or a pharmaceutical composition according to the invention, wherein the BRAF inhibitor is administered concurrently with the PD-1 axis binding antagonist;
A combination or a pharmaceutical composition according to the invention, wherein the BRAF inhibitor and the PD-1 axis binding antagonist are co-formulated;
A combination or a pharmaceutical composition according to the invention, wherein the BRAF inhibitor is administered sequentially with the PD-1 axis binding antagonist;
A combination or a pharmaceutical composition according to the invention, wherein the BRAF inhibitor is administered orally on a daily basis and wherein the PD-1 axis binding antagonist is administered once per week via intraveneous or subcutaneous injection;
A combination of a BRAF inhibitor and a PD-1 axis binding antagonist for use according to the invention, wherein the cancer is thyroid cancer, colorectal cancer, melanoma, brain cancer, leptomeningeal cancer or non-small cell lung cancer;
A combination of a BRAF inhibitor and a PD-1 axis binding antagonist for use according to the invention, wherein the cancer is associated with BRAF^{V600X} mutations;
A combination of a BRAF inhibitor and a PD-1 axis binding antagonist for use according to the invention, wherein the cancer is BRAF^{V600X} mutation-positive unresectable or metastatic cancer;
A combination BRAF inhibitor and a PD-1 axis binding antagonist for use according to the invention, wherein BRAF^{V600X} mutation is determined using a method comprising (a) performing PCR or sequencing on nucleic acid (e.g., DNA) extracted from a sample of the patient's tumour tissue and/or body fluid; and (b) determining expression of BRAF^{V600} in the sample;
A combination of a BRAF inhibitor and a PD-1 axis binding antagonist according to the invention or for use according to the invention, comprising one or more additional anticancer agents, in particular one or more anticancer agents selected from MEK inhibitors, MEK degraders, EGFR inhibitors, EGFR degraders, inhibitors of HER2 and/or HER3, degraders of HER2 and/or HER3, SHP2 inhibitors, SHP2 degraders, Axl inhibitors, Axl degraders, ALK inhibitors, ALK degraders, PI3K inhibitors, PI3K degraders, SOS1 inhibitors, SOS1 degraders, signal transduction patway inhibitors, checkpoint inhibitors, modulators of the apoptosis pathway, cytotoxic chemotherapeutics, angiogenesis-targeted therapies, immune-targeted agents, and antibody-drug conjugates;
A combination of a BRAF inhibitor and a PD-1 axis binding antagonist for use according to the invention, comprising in addition a MEK inhibitor;
A combination of a BRAF inhibitor and a PD-1 axis binding antagonist for use according to the invention, comprising in addition a MEK inhibitor selected from cobimetinib, binimetinib and trametinib;
A combination of a BRAF inhibitor and a PD-1 axis binding antagonist for use according to the invention, comprising in addition cobimetinib;
A combination of a BRAF inhibitor and a PD-1 axis binding antagonist according to the invention for use in the treatment or prophylaxis of cancer, wherein the BRAF inhibitor is (3R)-N-[2-cyano-4-fluoro-3-(3-methyl-4-oxo-quinazolin-6-yl)oxy-phenyl]-3-fluoro-pyrrolidine-1-sulfonamide or a pharmaceutically acceptable salt thereof;
A combination of a BRAF inhibitor and a PD-1 axis binding antagonist for use according to the invention, wherein the PD-1 axis binding antagonist is selected from cemiplimab (Libtayo^{®}), pembrolizumab (Keytruda^{®}), nivolumab (Opdivo^{®}), RN888 (PF-06801591), atezolizumab (Tecentriq^{®}), avelumab (Bavencio^{®}) and durvalumab (ImfinziTM);
A combination of a BRAF inhibitor and a PD-1 axis binding antagonist according to the invention for use according to the invention, wherein the PD-1 axis binding antagonist is atezolizumab (Tecentriq^{®});
A pharmaceutical composition as described herein, wherein the PD-1 axis binding anatgonist is a PD-1 binding antagonist selected from cemiplimab (Libtayo^{®}), pembrolizumab (Keytruda^{®}), nivolumab (Opdivo^{®}) and RN888 (PF-06801591);
A pharmaceutical composition as described herein, wherein the PD-1 axis binding anatgonist is a PD-L1 binding antagonist selected from atezolizumab (Tecentriq^{®}), avelumab (Bavencio^{®}) and durvalumab (ImfinziTM);
A pharmaceutical composition as described herein, wherein the PD-1 axis binding anatgonist is atezolizumab (Tecentriq^{®});
A pharmaceutical composition as described herein, wherein the compound of formula (I) is (3R)-N-[2-cyano-4-fluoro-3-(3-methyl-4-oxo-quinazolin-6-yl)oxy-phenyl]-3-fluoro-pyrrolidine-1-sulfonamide;
A pharmaceutical composition as described herein, wherein the compound of formula (I) is (3S)-N-[2-cyano-4-fluoro-3-(3-methyl-4-oxo-quinazolin-6-yl)oxy-phenyl]-3-fluoro-pyrrolidine-1-sulfonamide;
A pharmaceutical composition as described herein, for use in the treatment or prophylaxis of cancer, in particular thyroid cancer, colorectal cancer, melanoma, brain cancer, leptomeningeal cancer or non-small cell lung cancer;
A pharmaceutical composition as described herein, for use in the treatment or prophylaxis of cancer, in particular thyroid cancer, colorectal cancer, melanoma, brain cancer, leptomeningeal cancer or non-small cell lung cancer, wherein the BRAF inhibitor and the PD-1 axis binding antagonist are administered orally;
A pharmaceutical composition as described herein, for use in the treatment or prophylaxis of cancer, in particular thyroid cancer, colorectal cancer, melanoma, brain cancer or non-small cell lung cancer, wherein the first composition is administered concurrently with the second composition;
A pharmaceutical composition as described herein, for use in the treatment or prophylaxis of cancer, in particular thyroid cancer, colorectal cancer, melanoma, brain cancer, leptomeningeal cancer or non-small cell lung cancer, wherein the first and second compositions are co-formulated;
A pharmaceutical composition as described herein, for use in the treatment or prophylaxis of cancer, in particular thyroid cancer, colorectal cancer, melanoma, brain cancer, leptomeningeal cancer or non-small cell lung cancer, wherein the first composition is administered sequentially with the second composition;
A pharmaceutical composition as described herein, for use in the treatment or prophylaxis of cancer, in particular thyroid cancer, colorectal cancer, melanoma, brain cancer, leptomeningeal cancer or non-small cell lung cancer;
A pharmaceutical composition as described herein, for use in the treatment or prophylaxis of cancer, wherein the cancer is associated to BRAF mutations;
A pharmaceutical composition as described herein, for use in the treatment or prophylaxis of cancer, wherein the cancer is BRAF^{V600X} mutation-positive unresectable or metastatic cancer, in particular BRAF^{V600E} or BRAF^{V600K} mutation-positive unresectable or metastatic cancer;
A pharmaceutical composition as described herein, for use in the treatment or prophylaxis of cancer, in particular thyroid cancer, colorectal cancer, melanoma, brain cancer or non-small cell lung cancer, wherein BRAF^{V600X} mutation is determined using a method comprising (a) performing PCR or sequencing on nucleic acid (e.g., DNA) extracted from a sample of the patient's tumour tissue and/or body fluid; and (b) determining expression of BRAF^{V600} in the sample;
A certain embodiment of the invention relates to a pharmaceutical composition as described herein, for the use as a medicament in therapeutic and/or prophylactic treatment of brain metastases;
A certain embodiment of the invention relates to a pharmaceutical composition as described herein, for the use as a medicament in therapeutic and/or prophylactic treatment of brain metastases, wherein the primary tumour is melanoma or non-small cell lung cancer;
A certain embodiment of the invention relates to a pharmaceutical composition as described herein, for use in the treatment and/or prophylaxis cancer, in particular melanoma or non-small cell lung cancer, wherein the patient is treatment-naive regading targeted therapy;
A certain embodiment of the invention relates to a pharmaceutical composition as described herein, for use in the treatment and/or prophylaxis cancer, in particular melanoma or non-small cell lung cancer, wherein the patient is treatment-naive regading targeted therapy, and wherein the patient is checkpoint inhibitor treatment-experienced;
A certain embodiment of the invention relates to a pharmaceutical composition as described herein, for use in the treatment and/or prophylaxis cancer, in particular melanoma or non-small cell lung cancer, wherein the patient is treatment-experienced regarding targeted therapy, and wherein the patient is checkpoint inhibitor treatment-experienced;
A certain embodiment of the invention relates to a pharmaceutical composition as described herein, for use in the treatment and/or prophylaxis cancer, in particular melanoma or non-small cell lung cancer, wherein the cancer was previously treated by surgery;
A certain embodiment of the invention relates to a pharmaceutical composition as described herein, for use in the treatment and/or prophylaxis of cancer, in particular melanoma or non-small cell lung cancer, wherein the patient is treatment-naive regarding BRAF inhibitor treatment;
A certain embodiment of the invention relates to a pharmaceutical composition as described herein, for the use as a medicament in therapeutic and/or prophylactic treatment of BRAF inhibitor treatment-resistant tumour;
A certain embodiment of the invention relates to a pharmaceutical composition as described herein, for use in the treatment and/or prophylaxis of cancer, in particular melanoma, non-small cell lung cancer or leptomeningeal cancer, wherein the patient is treatment-naïve regarding MEK inhibitor treatment;
A certain embodiment of the invention relates to a pharmaceutical composition as described herein, for use in the treatment and/or prophylaxis of cancer, in particular melanoma or non-small cell lung cancer, wherein the patient is treatment-naïve regarding PD-1 axis binding anatgonist treatment;
A certain embodiment of the invention relates to a pharmaceutical composition as described herein, for the use as a medicament in therapeutic and/or prophylactic treatment of MEK inhibitor treatment-resistant tumour; and
A certain embodiment of the invention relates to a pharmaceutical composition as described herein, for the use as a medicament in therapeutic and/or prophylactic treatment of PD-1 axis binding anatgonist treatment-resistant tumour.
A certain embodiment of the invention relates to a pharmaceutical composition as described herein, for the use as a medicament in therapeutic and/or prophylactic treatment of cancer in a subject which was previously treated with a BRAF inhibitor selected from encorafenib, dabrafenib and encorafenib, and a MEK inhibitor selected from binimetinib, trametinib and cobimetinib;
A certain embodiment of the invention relates to a pharmaceutical composition as described herein, for the use as a medicament in therapeutic and/or prophylactic treatment of cancer in a subject which was previously treated with a BRAF inhibitor selected from encorafenib, dabrafenib and encorafenib, and a PD-1 axis binding anatgonist;
A certain embodiment of the invention relates to a pharmaceutical composition as described herein, for the use as a medicament in therapeutic and/or prophylactic treatment in a subject which was previously treated with encorafenib and binimetinib;
A certain embodiment of the invention relates to a pharmaceutical composition as described herein, for the use as a medicament in therapeutic and/or prophylactic treatment in a subject which was previously treated with dabarafenib and trametinib;
A certain embodiment of the invention relates to a pharmaceutical composition as described herein, for the use as a medicament in therapeutic and/or prophylactic treatment in a subject which was previously treated with vemurafenib and cobimetinib;
A certain embodiment of the invention relates to a pharmaceutical composition as described herein, for the use as a medicament in therapeutic and/or prophylactic treatment of cancer, in particular melanoma or non-small cell lung cancer, in a subject which was previously treated with a checkpoint inhibitor;
A certain embodiment of the invention relates to a pharmaceutical composition as described herein, for the use as a medicament in therapeutic and/or prophylactic treatment of cancer, in particular melanoma or non-small cell lung cancer, in a subject which was previously treated with a PD-1 axis binding antagonist;
A certain embodiment of the invention relates to a BRAF inhibitor and a PD-1 axis binding antagonist as described herein, for use in the therapeutic and/or prophylactic treatment of cancer, in particular melanoma or non-small cell lung cancer, wherein the patient is treatment-naive regarding targeted therapy;
A certain embodiment of the invention relates to a BRAF inhibitor and a PD-1 axis binding antagonist as described herein, for use in the therapeutic and/or prophylactic treatment of cancer, in particular melanoma or non-small cell lung cancer, wherein the patient is treatment-naive regarding targeted therapy, and wherein the patient is checkpoint inhibitor treatment-experienced;
A certain embodiment of the invention relates to a BRAF inhibitor and a PD-1 axis binding anatgonist as described herein, for use in the therapeutic and/or prophylactic treatment of cancer, wherein the patient is treatment-experienced regarding targeted therapy, and wherein the patient is checkpoint inhibitor treatment-experienced;
A certain embodiment of the invention relates to a BRAF inhibitor and a PD-1 axis binding anatgonist as described herein, for use in the therapeutic and/or prophylactic treatment of cancer, in particular melanoma or non-small cell lung cancer, wherein the patient is treatment-naive regarding BRAF inhibitor treatment;
A certain embodiment of the invention relates to a BRAF inhibitor and a PD-1 axis binding antagonist as described herein, for the use as a medicament in therapeutic and/or prophylactic treatment of BRAF inhibitor treatment-resistant tumour;
A certain embodiment of the invention relates to a BRAF inhibitor and a PD-1 axis binding antagonist as described herein, for use in the therapeutic and/or prophylactic treatment of cancer, in particular melanoma or non-small cell lung cancer, wherein the patient is treatment-naive regarding PD-1 axis binding antagonist treatment;
A certain embodiment of the invention relates to a BRAF inhibitor and a PD-1 axis binding antagonist treatment as described herein, for use in therapeutic and/or prophylactic treatment of PD-1 axis binding antagonist treatment-resistant tumour;
A certain embodiment of the invention relates to a BRAF inhibitor of formula (I) or a pharmaceutically acceptable salt or solvate thereof and a PD-1 axis binding antagonist treatment as described herein, for the use as a medicament in therapeutic and/or prophylactic treatment of cancer in a subject which was previously treated with a BRAF inhibitor selected from encorafenib, dabrafenib and encorafenib, and/or a MEK axis binding antagonist treatment selected from cobimetinib, binimetinib and trametinib;
A certain embodiment of the invention relates to a BRAF inhibitor of formula (I) or a pharmaceutically acceptable salt or solvate thereof and a PD-1 axis binding antagonist treatment as described herein, for the use as a medicament in therapeutic and/or prophylactic treatment in a subject which was previously treated with encorafenib and binimetinib;
A certain embodiment of the invention relates to a BRAF inhibitor of formula (I) or a pharmaceutically acceptable salt or solvate thereof and a PD-1 axis binding antagonist treatment as described herein, for the use as a medicament in therapeutic and/or prophylactic treatment in a subject which was previously treated with dabarafenib and trametinib;
A certain embodiment of the invention relates to a BRAF inhibitor of formula (I) or a pharmaceutically acceptable salt or solvate thereof and a PD-1 axis binding antagonist treatment as described herein, for the use as a medicament in therapeutic and/or prophylactic treatment in a subject which was previously treated with vemurafenib and cobimetinib;
A certain embodiment of the invention relates to a BRAF inhibitor formula (I) or a pharmaceutically acceptable salt or solvate thereof and a PD-1 axis binding antagonist treatment as described herein, for use in therapeutic and/or prophylactic treatment of cancer, in particular melanoma or non-small cell lung cancer, in a subject which was previously treated with a checkpoint inhibitor;

In one embodiment, the treated subject became refractory to said prior treatment as described herein; and
In one embodiment, the treated subject developed brain metastasis during said prior treatment as described herein.

A certain embodiment of the invention relates to a combination accrding to the invention or a pharmaceutical composition comprising a compound of formula (I) as described herein, or a pharmaceutically acceptable salt thereof, wherein at least one substituent comprises at least one radioisotope. Particular examples of radioisotopes are ²H, ³H, ¹³C, ¹⁴C and ¹⁸F.

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

If desired, racemic mixtures of the compound of the invention may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where an optically pure enantiomer is provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer of the compound. A chirally pure or chirally enriched compound may be prepared by chirally selective synthesis or by separation of enantiomers. The separation of enantiomers may be carried out on the final product or alternatively on a suitable intermediate.

In one embodiment, one or more additional anticancer agents is used in combination with a BRAF inhibitor and a MEK inhibitor as described herein, wherein the additional anticancer agents are selected from MEK inhibitors, MEK degraders, EGFR inhibitors, EGFR degraders, inhibitors of HER2 and/or HER3, degraders of HER2 and/or HER3, SHP2 inhibitors, SHP2 degraders, Axl inhibitors, Axl degraders, ALK inhibitors, ALK degraders, PI3K inhibitors, PI3K degraders, SOS1 inhibitors, SOS1 degraders, signal transduction patway inhibitors, checkpoint inhibitors, modulators of the apoptosis pathway, cytotoxic chemotherapeutics, angiogenesis-targeted therapies, immune-targeted agents, and antibody-drug conjugates.

In some embodiments, one of the additional anticancer agents is a MEK inhibitor. Non-limiting examples of MEK inhibitors include cobimetinib (Cotellic^{®}), binimetinib (Mektovi^{®}) and trametinib (Mekinist^{®}). Additional examples of MEK inhibitors are known in the art.

In some embodiments, one of the additional anticancer agents is an EGFR inhibitor. Non-limiting examples of EGFR inhibitors include cetuximab (Erbitux^{®}), panitumumab (Vectibix^{®}), osimertinib (merelectinib, Tagrisso^{®}), erlotinib (Tarceva^{®}), gefitinib (Iressa^{®}), necitumumab (PortrazzaTM), neratinib (Nerlynx^{®}), lapatinib (Tykerb^{®}), vandetanib (Caprelsa^{®}) and brigatinib (Alunbrig^{®}). Additional examples of EGFR inhibitors are known in the art. In some embodiments the EGFR inhibitor is an allosteric EGFR inhibitor.

In some embodiments, one of the additional anticancer agents is an inhibitor of HER2 and/or HER3. Non-limiting examples of HER2 and/or HER3 inhibitors include Iapatinib, canertinib, (E)-2-methoxy-N-(3-(4-(3-methyl-4-(6-methylpyridin-3-yloxy)phenylamino)quinazolin-6-yl)allyl)acetamide (GP-724714), sapitinib, 7-[[4-[(3-ethynylphenyl)amino]-7-methoxy-6-quinazolinyl]oxy]-N-hydroxy-heptanamide (CUDC-101), mubritinib, 6-[4-[(4-ethylpiperazin-1-yl)methyl]phenyl]-N-[(1R)-1-phenylethyl]-7H-pyrrolo[2,3-d]pyrimidin-4-amine (AEE788), irbinitinib (tucatinib), poziotinib, N-[4-[1-[4-(4-acetyl-1-piperazinyl)cyclohexyl]-4-amino-3-pyrazolo[3,4-d]pyrimidinyl]-2-methoxyphenyl]-1-methyl-2-indolecarboxamide (KIN001-111), 7-cyclopentyl-5-(4-phenoxyphenyl)-7H-pyrrolo[2,3-d]pyrimidin-4-ylamine (KIN001-051), 6,7-dimethoxy-N-(4-phenoxyphenyl)quinazolin-4-amine (KIN001-30), dasatinib, and bosutinib.

In some embodiments, one of the additional anticancer agents is an inhibitor of SHP2. Non-limiting examples of SHP2 inhibitors include 6-(4-amino-4-methylpiperidin-1-yl)-3-(2,3-dichlorophenyl)pyrazine-2-amine (SHP099), [3-[(3S,4S)-4-amino-3-methyl-2-oxa-8-azaspiro[4.5]decan-8-yl]-6-(2,3-dichlorophenyl)-5-methylpyrazin-2-yl]methanol (RMC-4550) RMC-4630, TNO155, and the compounds disclosed in WO 2015/107493, WO 2015/107494, WO 2015/107495, WO 2019/075265, PCT/U82019/056786 and PCT/182020/053019.

In some embodiments, one of the additional anticancer agents is a PI3K inhibitor. Non-limiting examples include buparlisib (BKM120), alpelisib (BYL719), samotolisib (LY3023414), 8-[(1R)-1-[(3,5-difluorophenyl)amino]ethyl]-N,N-dimethyl-2-(morpholin-4-yl)-4-oxo-4H-chromene-6-carboxamide (AZD8186), tenalisib (RP6530), voxtalisib hydrochloride (SAR-245409), gedatolisib (PF-05212384), panulisib (P-7170), taselisib (GDC-0032), trans-2-amino-8-[4-(2-hydroxyethoxy)cyclohexyl]-6-(6-methoxypyridin-3-yl)-4-methylpyrido[2,3-d]pyrimidin-7(8H)-one (PF-04691502), duvelisib (ABBV-954), N2-[4-oxo-4-[4-(4-oxo-8-phenyl-4H-1-benzopyran-2-yl)morpholin-4-ium-4-ylmethoxy]butyryl]-L-arginyl-glycyl-L-aspartyl-L-serine acetate (SF-1126), pictilisib (GDC-0941), 2-methyl-1-[2-methyl-3-(trifluoromethyl)benzyl]-6-(morpholin-4-yl)-1H-benzimidazole-4-carboxylic acid (GSK2636771), idelalisib (GS-1101), umbralisib tosylate (TGR-1202), pictilisib (GDC-0941), copanlisib hydrochloride (BAY 84-1236), dactolisib (BEZ-235), 1-(4-[5-[5-amino-6-(5-tert-butyl-1,3,4-oxadiazol-2-yl)pyrazin-2-yl]-1-ethyl-1-H-1,2,4-triazol-3-yl]piperidin-1-yl)-3-hydroxypropan-1-one (AZD-8835), 5-[6,6-dimethyl-4-(morpholin-4-yl)-8,9-dihydro-6H-[1,4]oxazino[4,3-e]purin-2-yl]pyrimidin-2-amine (GDC-0084) everolimus, rapamycin, perifosine, sirolimus and temsirolimus.

In some embodiments, one of the additional anticancer agents is an ALK inhibitor. Non-limiting examples include crizotinib (PF-02341066), ceritinib (LDK378), alectinib (alecensa), brigatinib (AP26113), lorlatinib (PF-6463922), ensartinib (X-396), entrectinib (RXDX-101), reprotectinib (TPX-0005), belizatinib (TSR-011), alkotinib (ZG-0418), foritinib (SAF-189), CEP-37440, TQ-B3139, PLB1003 and TPX-0131

In some embodiments, one of the additional anticancer agents is a checkpoint inhibitor. In some embodiments, the checkpoint inhibitor is a CTLA-4 inhibitor, a PD-1 binding antagonist or a PD-L1 binding antagonist. In some embodiments, the CTLA-4 inhibitor is ipilimumab (Yervoy^{®}) or tremelimumab (GP-675,206). In some embodiments, the PD-1 binding antagonist is selected from cemiplimab (Libtayo^{®}), pembrolizumab (Keytruda^{®}), nivolumab (Opdivo^{®}) and RN888 (PF-06801591). In some embodiments, the PD-L1 binding antagonist is selected from atezolizumab (Tecentriq^{®}), avelumab (Bavencio^{®}) and durvalumab (Imfinzi^{™}).

In some embodiments, one of the additional anticancer agents is an antibody-drug conjugate. Non-limiting examples of an antibody-drug conjugate include gemtuzumab ozogamicin (MylotargTM), inotuzumab ozogamicin (Besponsa^{®}), brentuximab vedotin (Adcetris^{®}), ado-trastuzumab emtansine (TDM-f; Kadcyla^{®}), mirvetuximab soravtansine (IMGN853) and anetumab ravtansine.

In some embodiments, one of the additional anticancer agents is an antibody such as bevacizumab (MvastiTM, Avastin^{®}), trastuzumab (Herceptin^{®}), avelumab (Bavencio^{®}), rituximab (MabTheraTM, Rituxan^{®}), edrecolomab (Panorex), daratumuab (Darzalex^{®}), olaratumab (LartruvoTM), ofatumumab (Arzerra^{®}), alemtuzumab (Campath^{®}), cetuximab (Erbitux^{®}), oregovomab, cemiplimab (Libtayo^{®}), pembrolizumab (Keytruda^{®}), dinutiximab (Unituxin^{®}), obinutuzumab (Gazyva^{®}), tremelimumab (GP-675,206), ramucirumab (Cyramza^{®}), ublituximab (TG-1101), panitumumab (Vectibix^{®}), elotuzumab (EmplicitiT'V'), necitumumab (PortrazzaT'V'), cirmtuzumab (UC-961), ibritumomab (Zevalin^{®}), isatuximab (SAR650984), nimotuzumab, fresolimumab (GC1008), Iirilumab (INN), mogamulizumab (Poteligeo^{®}), ficlatuzumab (AV-299), denosumab (Xgeva^{®}), ganitumab, urelumab, pidilizumab, amatuximab, blinatumomab (AMG103; Blincyto^{®}) or midostaurin (Rydapt).

In some embodiments, the isolated anti-PD-L1 antibody is glycosylated. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Removal of glycosylation sites form an antibody is conveniently accomplished by altering the amino acid sequence such that one of the above-described tripeptide sequences (for N-linked glycosylation sites) is removed. The alteration may be made by substitution of an asparagine, serine or threonine residue within the glycosylation site another amino acid residue (e.g., glycine, alanine or a conservative substitution).

In any of the embodiments herein, the isolated anti-PD-L1 antibody can bind to a human PD-L1, for example a human PD-L1 as shown in UniProtKB/Swiss-Prot Accession No.Q9NZQ7.1, or a variant thereof.

In a still further embodiment, the invention provides for a composition comprising an anti-PD-L1, an anti-PD-1, or an anti-PD-L2 antibody or antigen binding fragment thereof as provided herein and at least one pharmaceutically acceptable carrier. In some embodiments, the anti-PD-L1, anti-PD-1, or anti-PD-L2 antibody or antigen binding fragment thereof administered to the individual is a composition comprising one or more pharmaceutically acceptable carrier. Any of the pharmaceutically acceptable carriers described herein or known in the art may be used.

In some embodiments, the anti-PD-L1 antibody described herein is in a formulation comprising the antibody at an amount of about 60 mg/mL, histidine acetate in a concentration of about 20 mM, sucrose in a concentration of about 120 mM, and polysorbate (e.g., polysorbate 20) in a concentration of 0.04% (w/v), and the formulation has a pH of about 5.8. In some embodiments, the anti-PD-L1 antibody described herein is in a formulation comprising the antibody in an amount of about 125 mg/mL, histidine acetate in a concentration of about 20 mM, sucrose is in a concentration of about 240 mM, and polysorbate (e.g., polysorbate 20) in a concentration of 0.02% (w/v), and the formulation has a pH of about 5.5.

### Antibody Preparation

As described above, in some embodiments, the PD-1 binding antagonist is an antibody (e.g., an anti-PD-1 antibody, an anti-PD-L1 antibody, or an anti-PD-L2 antibody). The antibodies described herein may be prepared using techniques available in the art for generating antibodies, exemplary methods of which are described in more detail in the following sections. The antibody is directed against an antigen of interest. For example, the antibody may be directed against PD-1 (such as human PD-1), PD-L1 (such as human PD-L1), PD-L2 (such as human PD-L2). Preferably, the antigen is a biologically important polypeptide and administration of the antibody to a mammal suffering from a disorder can result in a therapeutic benefit in that mammal.

In certain embodiments, an antibody described herein has a dissociation constant (Kd) of 1µM, 150 nM, 100 nM, 50 nM, 10 nM, 1 nM, 0.1 nM, 0.01 nM, or 0.001 nM (e.g. 10-8 M or less, e.g. from 10-8 M to 10-13 M, *e.g.,* from 10-9 M to 10-13 M).

In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (125I)-labeled antigen in the presence of a titration series of unlabeled antigen, then capturing bound antigen with an anti-Fab antibody-coated plate (see, *e.g.,* Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [125I]-antigen are mixed with serial dilutions of a Fab of interest. The Fab of interest is then incubated overnight; however, the incubation may continue for a longer period (*e.g.,* about 65 hours) to ensure that equilibrium is reached. Thereafter, the mixtures are transferred to the capture plate for incubation at room temperature (*e.g.,* for one hour). The solution is then removed and the plate washed eight times with 0.1% polysorbate 20 (TWEEN-20^{®}) in PBS. When the plates have dried, 150 µl/well of scintillant (MICROSCINT-20 TM; Packard) is added, and the plates are counted on a TOPCOUNT TM gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE^{®}-2000 or a BIACORE^{®}-3000 (BIAcore, Inc., Piscataway, NJ) at 25°C with immobilized antigen CM5 chips at ~10 response units (RU). Briefly, carboxymethylated dextran biosensor chips (CM5, BIACORE, Inc.) are activated with *N*-ethyl-*N*'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25°C at a flow rate of approximately 25 µl/min. Association rates (kon) and dissociation rates (koff) are calculated using a simple one-to-one Langmuir binding model (BIACORE^{®} Evaluation Software version 3.2) by simultaneously fitting the association and dissociation sensorgrams. The equilibrium dissociation constant (Kd) is calculated as the ratio koff/kon. See, *e.g.,* Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO TM spectrophotometer (ThermoSpectronic) with a stirred cuvette.

### Antibody Fragments

In certain embodiments, an antibody described herein is an antibody fragment. Antibody fragments include, but are not limited to, Fab, Fab', Fab'-SH, F(ab')2, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1). Antibody fragments can be made by various techniques, including but not limited to proteolytic digestion of an intact antibody as well as production by recombinant host cells (e.g. *E. coli* or phage), as described herein.

### Chimeric and Humanized Antibodies

In certain embodiments, an antibody described herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof. In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall' Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### Human Antibodies

In certain embodiments, an antibody described herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSETM technology; U.S. Patent No. 5,770,429 describing HUMAB^{®} technology; U.S. Patent No. 7,041,870 describing K-M MOUSE^{®} technology, and U.S. Patent Application Publication No. US 2007/0061900, describing VELOCIMOUSE^{®} technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing humanhuman hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### Library-Derived Antibodies

Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as singlechain Fv (scFv) fragments or as Fab fragments. Libraries from immunized sources provide highaffinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227:381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360. Antibodies or antibody fragments isolated from human antibody libraries are considered human antibodies or human antibody fragments herein.

### Multispecific Antibodies

In certain embodiments, an antibody described herein is a multispecific antibody, e.g. a bispecific antibody. Multispecific antibodies are monoclonal antibodies that have binding specificities for at least two different sites. In some embodiments, the PD-1 axis component antagonist is multispecific. In one of the binding specificities is for a PD-1 axis component (e.g., PD-1, PD-L1, or PD-L2) and the other is for any other antigen. In some embodiments, one of the binding specificities is for IL-17 or IL-17R and the other is for any other antigen. In certain embodiments, bispecific antibodies may bind to two different epitopes of a PD-1 axis component (e.g., PD-1, PD-L1, or PD-L2), IL-17, or IL-17R. Bispecific antibodies can be prepared as full length antibodies or antibody fragments.

In some embodiments, one of the binding specificities is for a PD-1 axis component (e.g., PD-1, PD-L1, or PD-L2) and the other is for IL-17 or IL-17R. Provided herein are methods for treating or delaying progression of cancer in an individual comprising administering to the individual an effective amount of a multispecific antibody, wherein the multispecific antibody comprises a first binding specificity for a PD-1 axis component (e.g., PD-1, PD-L1, or PD-L2) and a second binding specificity for IL-17 or IL-17R. In some embodiments, a multispecific antibody may be made by any of the techniques described herein and below.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multispecific antibodies may also be made by engineering electrostatic steering effects for making antibody Fc-heterodimeric molecules (WO 2009/089004A1); crosslinking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see, e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to a PD-1 axis component (e.g., PD-1, PD-L1, or PD-L2), IL-17, or IL-17R as well as another, different antigen (see, US 2008/0069820, for example).

### Nucleic Acid sequences, vectors and methods of production

Polynucleotides encoding a PD1 axis binding antagonist, e.g., antibodies, may be used for production of the PD1 axis binding antagonists described herein. The PD1 axis binding antagonists used according to the the invention may be expressed as a single polynucleotide that encodes the entire bispecific antigen binding molecule or as multiple (e.g., two or more) polynucleotides that are co-expressed. Polypeptides encoded by polynucleotides that are co-expressed may associate through, e.g., disulfide bonds or other means to form a functional PD1 axis binding antagonist antibody. For example, the light chain portion of a Fab fragment may be encoded by a separate polynucleotide from the portion of the bispecific antibody comprising the heavy chain portion of the Fab fragment, an Fc domain subunit and optionally (part of) another Fab fragment. When co-expressed, the heavy chain polypeptides will associate with the light chain polypeptides to form the Fab fragment. In another example, the portion of the PD-1 axis binding antagonist antigen binding portion provided therein comprising one of the two Fc domain subunits and optionally (part of) one or more Fab fragments could be encoded by a separate polynucleotide from the portion of the bispecific antibody provided therein comprising the other of the two Fc domain subunits and optionally (part of) a Fab fragment. When co-expressed, the Fc domain subunits will associate to form the Fc domain.

In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

### Antibody Variants

In certain embodiments, amino acid sequence variants of the PD-1 axis binding antagonist antibodies are contemplated, in addition to those described above. For example, it may be desirable to improve the binding affinity and/or other biological properties of the antibodies. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### Substitution, Insertion, and Deletion Variants

In certain embodiments, variants having one or more amino acid substitutions are provided. Sites of interest for substitutional mutagenesis include the HVRs and FRs. Conservative substitutions are shown in Table B under the heading of "conservative substitutions." More substantial changes are provided in Table B under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE B**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody used with the invention comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in a bispecific antibody or an antibody binding to DR5 of the invention may be made in order to create antibody variants with certain improved properties.

In one embodiment, bispecific antibody variants or variants of antibodies are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibody variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### Cysteine engineered antibody variants

In certain embodiments, it may be desirable to create cysteine engineered antibodies, e.g., THIOMABS, in which one or more residues of the antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### Recombinant Methods and Compositions

Antibodies of the invention may be obtained, for example, by solid-state peptide synthesis (e.g. Merrifield solid phase synthesis) or recombinant production. For recombinant production one or more polynucleotide encoding the antibodies (or fragments), e.g., as described above, is isolated and inserted into one or more vectors for further cloning and/or expression in a host cell. Such polynucleotide may be readily isolated and sequenced using conventional procedures. In one embodiment a vector, preferably an expression vector, comprising one or more of the polynucleotides of the invention is provided. Methods which are well known to those skilled in the art can be used to construct expression vectors containing the coding sequence of an antibody along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding an antibody (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the antibody, or variant or derivative thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells.

Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the antibody is desired, DNA encoding a signal sequence may be placed upstream of the nucleic acid encoding an antibody of the invention or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, *e.g.* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the antibody may be included within or at the ends of the antibody (fragment) encoding polynucleotide.

In a further embodiment, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one such embodiment a host cell comprises (e.g. has been transformed or transfected with) a vector comprising a polynucleotide that encodes an antibody of the invention or a part thereof. As used herein, the term "host cell" refers to any kind of cellular system which can be engineered to generate the antibody, e.g., anti-PD-1 antibodies, anti-PD-L1 antibodies, and anti-PD-L2 antibodies of the invention or fragments thereof. Host cells suitable for replicating and for supporting expression of antibodies of the invention are well known in the art. Such cells may be transfected or transduced as appropriate with the particular expression vector and large quantities of vector containing cells can be grown for seeding large scale fermenters to obtain sufficient quantities of the antibodies for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES^{™} technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr⁻ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain. Any animal species of antibody, antibody fragment, antigen binding domain or variable region can be used in the antibodies used according to the invention. Non-limiting antibodies, antibody fragments, antigen binding domains or variable regions useful in the present invention can be of murine, primate, or human origin. If the antibody is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall' Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain embodiments, the antigen binding moieties useful in the present invention are engineered to have enhanced binding affinity according to, for example, the methods disclosed in U.S. Pat. Appl. Publ. No. 2004/0132066. The ability of the antibody of the invention to bind to a specific antigenic determinant can be measured either through an enzyme-linked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen. In certain embodiments, such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen (e.g. PD-1) is incubated in a solution comprising a first labeled antibody that binds to the antigen (e.g. V9 antibody, described in US 6,054,297) and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

Antibodies prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the bispecific antibody or the antibody binding to DR5 binds. For example, for affinity chromatography purification of bispecific antibodies of the invention, a matrix with protein A or protein G may be used. Sequential Protein A or G affinity chromatography and size exclusion chromatography can be used to isolate a bispecific antibody essentially as described in the Examples. The purity of the bispecific antibody or the antibody binding to DR5 can be determined by any of a variety of well-known analytical methods including gel electrophoresis, high pressure liquid chromatography, and the like.

### Assays

Antibodies, e.g., anti-PD-1 axis binding antagonist antibodies provided herein may be identified, screened for, or characterized for their physical/chemical properties and/or biological activities by various assays known in the art.

### Affinity assays

The affinity of the antibodies, e.g., anti-PD-1 axis binding antagonist antibodies provided herein for their respective antigen, e.g., PD-1, PD-L1, can be determined in accordance with the methods set forth in the Examples by surface plasmon resonance (SPR), using standard instrumentation such as a BIAcore instrument (GE Healthcare), and receptors or target proteins such as may be obtained by recombinant expression. Alternatively, binding of antibodies provided therein to their respective antigen may be evaluated using cell lines expressing the particular receptor or target antigen, for example by flow cytometry (FACS).

K_{D} may be measured by surface plasmon resonance using a BIACORE^{®} T100 machine (GE Healthcare) at 25°C. To analyze the interaction between the Fc-portion and Fc receptors, His-tagged recombinant Fc-receptor is captured by an anti-Penta His antibody (Qiagen) ("Penta His") immobilized on CM5 chips and the bispecific constructs are used as analytes. Briefly, carboxymethylated dextran biosensor chips (CM5, GE Healthcare) are activated with N-ethyl-N'-(3-dimethylaminopropyl)-carbodiimide hydrochloride (EDC) and N-hydroxysuccinimide (NHS) according to the supplier's instructions. Anti Penta-His antibody ("Penta His") is diluted with 10 mM sodium acetate, pH 5.0, to 40 µg/ml before injection at a flow rate of 5 µl/min to achieve approximately 6500 response units (RU) of coupled protein. Following the injection of the ligand, 1 M ethanolamine is injected to block unreacted groups. Subsequently the Fc-receptor is captured for 60 s at 4 or 10 nM. For kinetic measurements, four-fold serial dilutions of the bispecific construct (range between 500 nM and 4000 nM) are injected in HBS-EP (GE Healthcare, 10 mM HEPES, 150 mM NaCl, 3 mM EDTA, 0.05 % Surfactant P20, pH 7.4) at 25 °C at a flow rate of 30 µl/min for 120 s.

To determine the affinity to the target antigen, bispecific constructs are captured by an anti human Fab specific antibody (GE Healthcare) that is immobilized on an activated CM5-sensor chip surface as described for the anti Penta-His antibody ("Penta His"). The final amount of coupled protein is is approximately 12000 RU. The bispecific constructs are captured for 90 s at 300 nM. The target antigens are passed through the flow cells for 180 s at a concentration range from 250 to 1000 nM with a flowrate of 30 µl/min. The dissociation is monitored for 180 s.

Bulk refractive index differences are corrected for by subtracting the response obtained on reference flow cell. The steady state response was used to derive the dissociation constant K_{D} by non-linear curve fitting of the Langmuir binding isotherm. Association rates (kₒₙ) and dissociation rates (K_{off}) are calculated using a simple one-to-one Langmuir binding model (BIACORE^{®} T100 Evaluation Software version 1.1.1) by simultaneously fitting the association and dissociation sensorgrams. The equilibrium dissociation constant (K_{D}) is calculated as the ratio k_{off}/kₒₙ. See, e.g., Chen et al., J Mol Biol 293, 865-881 (1999).

### Binding assays and other assays

In one aspect, an antibodies, e.g., anti-PD-1 axis binding antagonist antibodies of the invention is tested for its antigen binding activity, e.g., by known methods such as ELISA, Western blot, etc.

In another aspect, competition assays may be used to identify an antibody or fragment that competes with a specific reference antibody for binding to the respective antigens. In certain embodiments, such a competing antibody binds to the same epitope (e.g., a linear or a conformational epitope) that is bound by a specific reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). Further methods are described in the example section.

### Activity assays

In one aspect, assays are provided for identifying antibodies, e.g., anti-PD-1 axis binding antagonist antibodies provided herein having biological activity. Biological activity may include, e.g., inducing DNA fragmentation, induction of apoptosis and lysis of targeted cells. Antibodies having such biological activity *in vivo* and/or *in vitro* are also provided.

In certain embodiments, an antibody of the invention is tested for such biological activity. Assays for detecting cell lysis (e.g. by measurement of LDH release) or apoptosis (e.g. using the TUNEL assay) are well known in the art. Assays for measuring ADCC or CDC are also described in WO 2004/065540 (see Example 1 therein).

### Pharmaceutical Formulations

Pharmaceutical formulations of antibodies, e.g., anti-PD-1 axis binding antagonist antibodies as described herein are prepared by mixing such antibody having the desired degree of purity with one or more optional pharmaceutically acceptable carriers *(*Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g.* films, or microcapsules.

The formulations to be used for *in vivo* administration are generally sterile. Sterility may be readily accomplished, e.g., by filtration through sterile filtration membranes.

An antibody can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Another embodiment of the invention provides a pharmaceutical composition containing one or more compositions, wherein each composition contains one or more compounds for use according to the invention and one or more therapeutically inert carriers, diluents or excipients, as well as a method to prepare such a pharmaceutical compositions. In one example, the compound of formula (I) may be formulated by mixing at ambient temperature at the appropriate pH, and at the desired degree of purity, with physiologically acceptable carriers, i.e., carriers that are nontoxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compound of formula (I) is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

As used herein, a "pharmaceutically acceptable carrier" or "pharmaceutically acceptable excipient" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

Pharmaceutical compositions can be obtained by processing the BRAF inhibitor as described herein with pharmaceutically acceptable, inorganic or organic carriers or excipients. Lactose, corn starch or derivatives thereof, talc, stearic acids or it's salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semiliquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Pharmaceutical compositions of a BRAF inhibitor, alone or in combination, can be prepared for storage by mixing the active ingredient having the desired degree of purity with optional pharmaceutically acceptable carriers, excipients or stabilizers (Remington's Pharmaceutical Sciences 16th edition, Osol, A. (ed.) (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEENTM, PLURONICSTM or polyethylene glycol (PEG).

Pharmaceutical compositions of a BRAF inhibitor include those suitable for oral, nasal, topical (including buccal and sublingual), rectal, vaginal and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a BRAF inhibitor or a PD-1 axis binding anatgonist treatment which produces a therapeutic effect. Generally, out of one hundred percent, this amount will range from about 1 percent to about 90 percent of active ingredient, preferably from about 5 percent to about 70 percent, most preferably from about 10 percent to about 30 percent. Methods of preparing these compositions include the step of bringing into association a BRAF inhibitor with the carrier and, optionally, one or more accessory ingredients. In general, the pharmaceutical compositions can be prepared by uniformaly and intimately bringing into association a BRAF inbitor with liquid carriers, or finely divided solid carriers, or both, and then, if necessary, shaping the product. Pharmaceutical compositions suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a BRAF inhibitor as an active ingredient. A BRAF inhibitor may also be administered as a bolus, electuary or paste.

In one embodiment of the invention, a BRAF inhibitor and a PD-1 axis binding anatgonist treatment are formulated into two separate pharmaceutical compositions.

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences, 16th edition, Osol, A. (ed.) (1980).

The formulations to be used for *in vivo* administration must be sterile. This can be readily accomplished by filtration through sterile filtration membranes.

The dosage can vary within wide limits and will, of course, have to be adjusted to the individual requirements in each particular case. In the case of oral administration the dosage for adults can vary from about 0.01 mg to about 1000 mg per day of a compound of general formula (I) or of the corresponding amount of a pharmaceutically acceptable solvatethereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical compositions conveniently contain about 1-500 mg, particularly 1-100 mg, of a compound of formula (I).

Examples of compositions according to the invention are:

### Example A

Tablets of the following composition are manufactured in the usual manner:

**Table 3: possible tablet composition**

| ingredient | mg/tablet | | | |
|---|---|---|---|---|
| | 5 | 25 | 100 | 500 |
| Compound of formula (I) | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 4: possible capsule ingredient composition**

| ingredient | mg/capsule | | | |
|---|---|---|---|---|
| | 5 | 25 | 100 | 500 |
| Compound of formula (I) | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula (I), lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 5: possible soft gelatin capsule ingredient composition**

| ingredient | mg/capsule |
|---|---|
| Compound of formula (I) | 5 |
| Yellow wax | 8 |
| Hydrogenated Soya bean oil | 8 |
| Partially hydrogenated plant oils | 34 |
| Soya bean oil | 110 |
| Total | 165 |

**Table 6: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula (I) is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 7: possible suppository composition**

| ingredient | mg/supp. |
|---|---|
| Compound of formula (I) | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula (I) is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 8: possible injection solution composition**

| ingredient | mg/injection solution. |
|---|---|
| Compound of formula (I) | 3 |
| Polyethylene Glycol 400 | 150 |
| acetic acid | q.s. ad pH 5.0 |
| water for injection solutions | ad 1.0 ml |

### Manufacturing Procedure

The compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 9: possible sachet composition**

| ingredient | mg/sachet |
|---|---|
| Compound of formula (I) | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula (I) is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Examples

### Abbreviations

CAS = chemical abstracts service; DCM = dichloromethane; DIPEA = N,N-diisopropylethylamine; DMF = dimethylformamide; DMSO = dimethyl sulfoxide; DNA = deoxyribonucleic acid; EDC·HCl = 1-(3-dimethylaminopropyl)-3-ethylcarbodiimide hydrochloride; ESI = electrospray ionization; EtOAc = ethyl acetate; HOOBt = 3,4-dihydro-3-hydroxy-4-oxo-1,2,3-benzotriazine; LC-MS/MS = liquid chromatography-MS/MS; MeOH = methanol; MS = mass spectrometry; NMP = N-methyl-2-pyrrolidone; PCR = polymerase chain reaction; rt = room temperature; SFC = supercritical fluid chromatography; THF = tetrahydrofuran.

The following examples and figures are provided to illustrate the invention and have no limiting character.

### Test agents

(3*R*)-*N-*[2-cyano-4-fluoro-3-(3-methyl-4-oxo-quinazolin-6-yl)oxy-phenyl]-3-fluoro-pyrrolidine-1-sulfonamide (herein referred to as Compound Ia) was provided as a powder from Roche, Basel, Switzerland and resuspended prior to use. Anti-mouse PD-1 (CD279; herein referrd to as anti-mPD-1) was purchased from BioXCell (clone RMP1-14, catalog #: BP0146).

### Cell line and culture conditions

Cell lines were obtained from ATCC, maintained in humidified incubators at 5% CO2 in standard conditions and passaged twice a week. Culture conditions are reported in the following table:

| Cell line | catalog#/ origin | culture condition |
|---|---|---|
| A375 | ATCC # CRL-1619 | Dulbecco's Modified Eagle's Medium, high glucose, GlutaMAX (DMEM GIBCO #10566016), 10% Fetal Bovine Serum (FBS, GIBCO #10270-106) |
| A375-NRAS | ATCC # CRL-1619IG-2 | Dulbecco's Modified Eagle's Medium, high glucose, GlutaMAX (DMEM GIBCO #10566016), 10% Fetal Bovine Serum (FBS, GIBCO #10270-106) |
| HCT116 | ATCC # CCL-247 | Dulbecco's Modified Eagle's Medium, high glucose, GlutaMAX (DMEM GIBCO #10566016), 10% Fetal Bovine Serum (FBS, GIBCO #10270-106) |
| YUMM1.7 | ATCC # CRL-3362 | Dulbecco's Modified Eagle's Medium: F12 (DMEM F12 GIBCO 30-2006), 10% Fetal Bovine Serum (FBS, GIBCO #10270-106), 1:100 addition of Non Essential Amminoacids (Gibco Cat# 11140050) |

The YUMM1.7 cell line was originally derived from a genetically engeneered mouse model of melanoma with the alleles: Tyrosinase:CreERT2 (to activate CRE recombinase expression restricted to cells expressing the tyrosinase gene; a gene with expression restricted to the melanocytic compartment), BRAF V600E/WT (BRAFV600E is conditionally activated only in cells expressing the CRE recombinase), PTEN -/-, CDKN2A -/- (PTEN and CDKN2A are conditionaly inactivated only in cells expressing the CRE recombinase). The murine cells derived from these tumors engraft in immunocomptent CBL/6 mice allowing to study the interaction with the immune compartment. The model is originally described in Meeth K, et al. The YUMM lines: a series of congenic mouse melanoma cell lines with defined genetic alterations. Pigment Cell Melanoma Res 29(5): 590-597, 2016. PubMed: 27287723.

### Animals:

In the in vivo studies, female mice 7-9 weeks old (at experiment initiation) were purchased from Charles River Laboratories. The strain utilized in the experiments was C57BL/6.

For xenograft establishment cells were suspended in a media composed of 50% Matrigel and 50% Hank's balanced Salt Solution and injected subcutaneously on the right flanks. When tumor volume reached around 100 mm³ mice were randomized prior treatment.

In the in vivo studies, female mice 7-9 weeks old (at experiment initiation) of the immunocompentent strain C57BL/6 were utilized. The experiment was performed at the Contract Research Organization (CRO) Covance that sourced mice, performed tumor establishment, drug treatment and tumor monitoring.

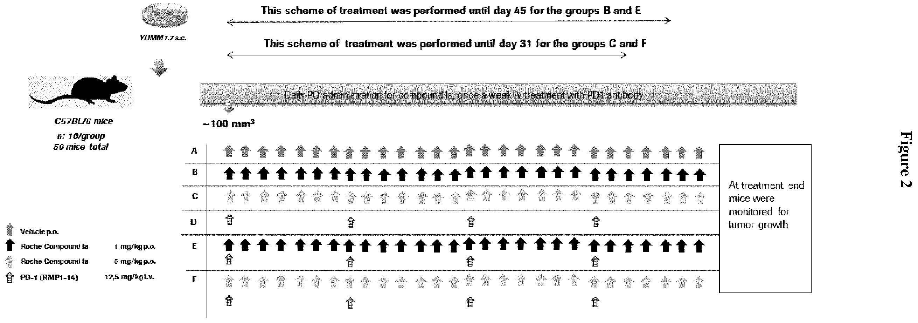

For xenograft establishment YUMM1.7 cells were suspended in a media composed of 50% Matrigel and 50% Hank's balanced Salt Solution and injected subcutaneously on the right flanks. When tumor volume reached around 100 mm³ mice were randomized prior treatment. Mice were treated according to the scheme reported in Figure 2.

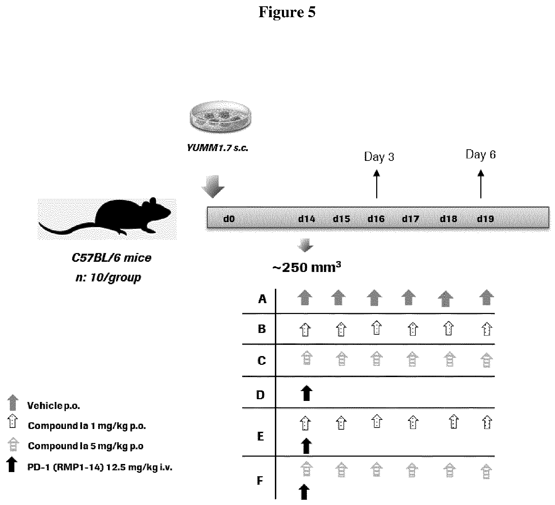

For the immunohistochemical analysis, female C57/BL6 mice were injected subQ with YUMM1.7 cells. When tumors reached around 250 mm3 (day 14) mice were treated with either Compound Ia at 1 or 5 mg/kg daily (oral administration), with anti-mouse PD1 at 12.5 mg/kg once (single intraveneous administration) or the combination of Compound Ia and anti-mPD1. Mice were treated according to the scheme reported in Figure 5. Mice were sacrificed and tumors collected upon 3 or five days from the beginning of the treatment and tumors were either digested and utilized for cytofluorimetric analysis of fixed and paraffin embedded. Cell suspension was incubated with (1:1000) Zombie Aqua^{™} dye (Biologend) for discrimination of alive and death cells and cytofluorimetric analysis was performed by staining cells with PE-labelled anti mouse CD45 or anti mouse CD3 (Clone SP-7 Abcam). For paraffin embedded tumors, samples were stained with anti-mouse CD45 (clone 30-F11 biolegend USA) according to standard procedure (Cancer Res. 2015 Mar 15;75(6):1091-101).

### Examples

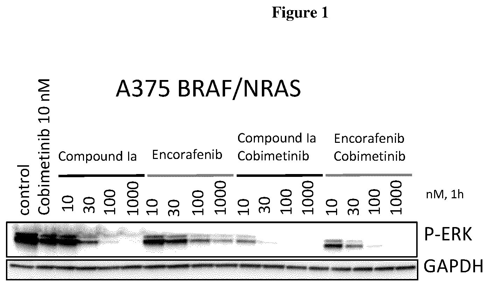

The Compound Ia is a novel paradox breaker BRAFi which was shown in Figure 1 and using the cell line A375 harboring the mutations BRAF V600E and NRAS Q61K; this cell line serves as model of resistance to first generation BRAFi and BRAF/MEKi combination.

Results evidence that Compound Ia triggers potent P-ERK inhibition in this cell line model which was superior to that achieved by Encorafenib as single agent. Moreover, combination with the MEKi cobimetinib at a fixed dose of 10 nM triggered a superior P-ERK reduction compared to the activity achieved by encorafenib/binimetinib.

### Example 1

Mice were implanted with the cell line YUMM1.7 harboring the BRAF V600E mutation. Upon tumor establishment (100mm3) mice were randomized and received orally (PO) once per day (QD) either Compound Ia (1 mg/kg or 5 mg/kg), mouse anti PD-1 antibody through IV injection, once a week or the respective combinations of PD-1 and Compound Ia at 1 or 5 mg/kg. The treatment was performed until from day 11 (treatment start upon randomization) to day 31 for the groups with Compound Ia 5mg/kg and combination PD-1/Compound Ia 5 mg/kg or from day 11 to day 45 for the groups with Compound Ia 1mg/kg and combination PD-1/Compound Ia 1 mg/kg. Upon treatment conclusion mice were monitored until day 58 for signs of tumor relapse. Results reported in figures 3 and 4 evidenced that the combination of Compound Ia and PD-1 manifests a strong impact on the number of mice showing disease relapse.

### Example 2

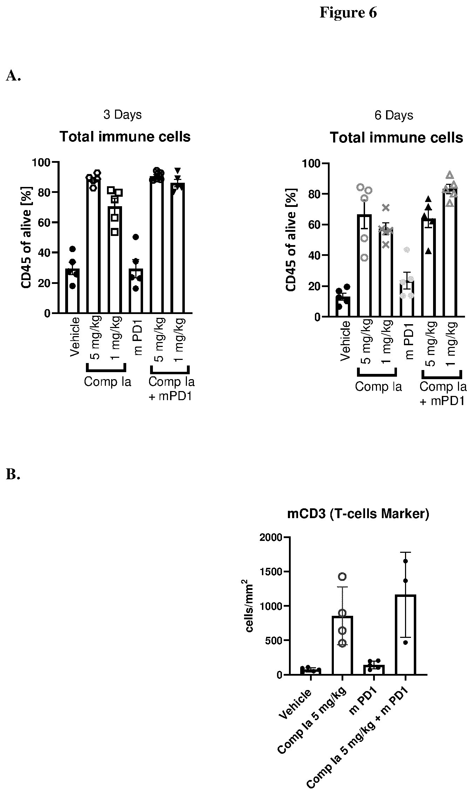

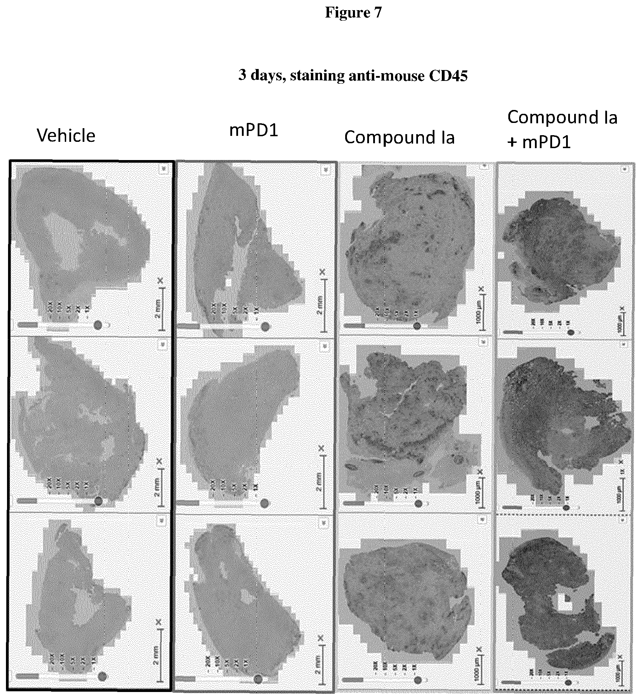

Results reported in Figures 6 and 7 clearly support the efficacy of a combination of Compound Ia with a PD-1 axis binding antagonist. In these experiments the treatment with Compound Ia is driving a robust antitumor response and thus triggers recruitment of inflammatory infiltrate, as observed in flow cytometry and IHC data. The activation of the immune system by the Compound Ia supports a strong effect when combining the Compound Ia with a PD-1 axis binding antagonist. In addition, increased release of tumoral antigens can promote an additional efficacy of the PD-1 axis blockage.

### Articles of Manufacture

Further disclosed is an article of manufacture containing materials useful for the treatment, prevention and/or diagnosis of the disorders described above. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a bispecific antibody and an additional active agent is the further chemotherapeutic agent as described herein. The label or package insert indicates that the composition is used for treating the condition of choice. Moreover, the article of manufacture may comprise (a) a first container with a composition contained therein, wherein the composition comprises a bispecific antibody; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Amino acid sequences of exemplary embodiments

### Exemplary anti-PD-1 and anti-PD-L1 antagonist sequences

| **Description** | **Sequence** | **Seq ID No** |
|---|---|---|
| anti-PD-L1 antibody | QVQLVESGGGVVQPGRSLRLDCKASGITFSNSGMHWVRQAP GKGLEWVAVIWYDGSKRYYADSVKGRFTISRDNSKNTLFLQ MNSLRAEDTAVYYCATNDDYWGQGTLVTVSSASTKGPSVFP LAPCSRSTSESTAALGCLVKDYFPEPVTVSWNSGALTSGVHTF PAVLQSSGLYSLSSVVTVPSSSLGTKTYTCNVDHKPSNTKVDK RVESKYGPPCPPCPAPEFLGGPSVFLFPPKPKDTLMISRTPEVT CVVVDVSQEDPEVQFNWYVDGVEVHNAKTKPREEQFNSTYR VVSVLTVLHQDWLNGKEYKCKVSNKGLPSSIEKTISKAKGQP REPQVYTLPPSQEEMTKNQVSLTCLVKGFYPSDIAVEWESNG QPENNYKTTPPVLDSDGSFFLYSRLTVDKSRWQEGNVFSCSV MHEALHNHYTQKSLSLSLGK | 1 |
| heavy chain | | |
| anti-PD-L1 antibody | EIVLTQSPATLSLSPGERATLSCRASQSVSSYLAWYQQKPGQA PRLLIYDASNRATGIPARFSGSGSGTDFTLTISSLEPEDFAVYYC QQSSNWPRTFGQGTKVEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC | 2 |
| light chain | | |
| anti-PD-L1 antibody | QVQLVQSGVEVKKPGASVKVSCKASGYTFTNYYMYWVRQA PGQGLEWMGGINPSNGGTNFNEKFKNRVTLTTDSSTTTAYME LKSLQFDDTAVYYCARRDYRFDMGFDYWGQGTTVTVSSAST KGPSVFPLAPCSRSTSESTAALGCLVKDYFPEPVTVSWNSGAL TSGVHTFPAVLQSSGLYSLSSVVTVPSSSLGTKTYTCNVDHKP SNTKVDKRVESKYGPPCPPCPAPEFLGGPSVFLFPPKPKDTLMI | 3 |
| heavy chain | | |
| | SRTPEVTCVVVDVSQEDPEVQFNWYVDGVEVHNAKTKPREE QFNSTYRVVSVLTVLHQDWLNGKEYKCKVSNKGLPSSIEKTI SKAKGQPREPQVYTLPPSQEEMTKNQVSLTCLVKGFYPSDIA VEWESNGQPENNYKTTPPVLDSDGSFFLYSRLTVDKSRWQEG NVFSCSVMHEALHNHYTQKSLSLSLGK | |
| anti-PD-L1 antibody | EIVLTQSPATLSLSPGERATLSCRASKGVSTSGYSYLHWYQQK PGQAPRLLIYLASYLESGVPARFSGSGSGTDFTLTISSLEPEDFA VYYCQHSRDLPLTFGGGTKVEIKRTVAAPSVFIFPPSDEQLKS GTASVVCLLNNFYPREAKVQWKVDNALQSGNSQESVTEQDS KDSTYSLSSTLTLSKADYEKHKVYACEVTHQGLSSPVT KSFNRGEC | 4 |
| light chain | | |
| anti-PD-L1 antibody | EVQLVESGGGLVQPGGSLRLSCAASGFTFSDSWIHWVRQAPG KGLEWVAWISPYGGSTYYADSVKGRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTLM ISRTPEVTCVVVDVSHEDPEVKFNWYVDGVEVHNAKTKPREE QYASTYRVVSVLTVLHQDWLNGKEYKCKVSNKALPAPIEKTI SKAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPG | 5 |
| heavy chain | | |
| anti-PD-L1 antibody | DIQMTQSPSSLSASVGDRVTITCRASQDVSTAVAWYQQKPGK APKLLIYSASFLYSGVPSRFSGSGSGTDFTLTISSLQPEDFATYY CQQYLYHPATFGQGTKVEIKRTVAAPSVFIFPPSDEQLKSGTA SVVCLLNNFYPREAKVQWKVDNALQSGNSQESVTEQDSKDS TYSLSSTLTLSKADYEKHKVYACEVTHQGLSSPVTKSFNRGE C | 6 |
| light chain | | |
| anti-PD-L1 antibody | | 7 |
| VH | | |
| anti-PD-L1 antibody | | 8 |
| VH | | |
| anti-PD-L1 antibody | DIQMTQSPSSLSASVGDRVTITCRASQDVSTAVAWYQQKPGK APKLLIYSASFLYSGVPSRFSGSGSGTDFTLTISSLQPEDFATYY CQQYLYHPATFGQGTKVEIKR | 9 |
| VL | | |
| HVR-H1 | GFTFSDSWIH | 10 |
| HVR-H2 | AWISPYGGSTYYADSVKG | 11 |
| HVR-H3 | RHWPGGFDY | 12 |
| HVR-L1 | RASQDVSTAVA | 13 |
| HVR-L2 | SASFLYS | 14 |
| HVR-L3 | QQYLYHPAT | 15 |

## Claims

1. A combination of a BRAF inhibitor and a PD-1 axis binding antagonist wherein the BRAF inhibitor is a compound of formula (I) 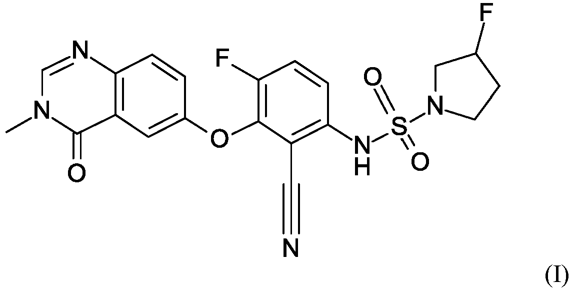 or a pharmaceutically acceptable salt or solvate thereof.

2. A combination of a BRAF inhibitor and a PD-1 axis binding antagonist according to claim 1, wherein the compound of formula (I) is (3R)-N-[2-cyano-4-fluoro-3-(3-methyl-4-oxo-quinazolin-6-yl)oxy-phenyl]-3-fluoro-pyrrolidine-1-sulfonamide.

3. A combination of a BRAF inhibitor and a PD-1 axis binding antagonist according to claim 1 or 2,wherein the PD-1 axis binding antagonist is selected from the group consisting of a PD-1 binding antagonist, a PD-Ll binding antagonist and a PD-L2 binding antagonist.

4. A combination of a BRAF inhibitor and a PD-1 axis binding antagonist according to any one of claims 1 to 3, wherein the PD-1 axis binding antagonist is a PD-1 binding antagonist.

5. A combination of a BRAF inhibitor and a PD-1 axis binding antagonist according to any one of claims 1 to 3, wherein the PD-1 axis binding antagonist is selected from cemiplimab (Libtayo^{®}), pembrolizumab (Keytruda^{®}), nivolumab (Opdivo^{®}), RN888 (PF-06801591), atezolizumab (Tecentriq^{®}), avelumab (Bavencio^{®}) and durvalumab (ImfinziTM).

6. A combination of a BRAF inhibitor and a PD-1 axis binding antagonist according to any one of claims 1 to 4, wherein the PD-1 axis binding antagonist is atezolizumab (Tecentriq^{®}).

7. A combination of a BRAF inhibitor and a PD-1 axis binding antagonist according to any one of claims 1 to 6, for use as a medicament.

8. A combination of a BRAF inhibitor and a PD-1 axis binding antagonist according to any one of claims 1 to 6, for use in the therapeutic and/or prophylactic treatment of cancer.

9. A pharmaceutical composition comprising a combination of a BRAF inhibitor and a PD-1 axis binding antagonist according to any one of claims 1 to 6 and one or more pharmaceutically acceptable excipients.

10. A combination for use according to any one of claims 7 to 8, wherein the BRAF inhibitor is administered orally and the PD-1 axis binding antagonist is administered by injection, such as intravenous or subcutaneous injection.

11. A combination for use according to any one of claims 7 to 8 or a pharmaceutical composition according to claim 9, wherein the BRAF inhibitor is administered concurrently with the PD-1 axis binding antagonist.

12. A combination for use according to any one of claims 7 to 8, wherein the BRAF inhibitor is administered sequentially with the PD-1 axis binding antagonist.

13. A combination of a BRAF inhibitor and a PD-1 axis binding antagonist for use according to claim 8, wherein the cancer is thyroid cancer, colorectal cancer, melanoma, brain cancer, leptomeningeal cancer or non-small cell lung cancer.

14. A combination of a BRAF inhibitor and a PD-1 axis binding antagonist for use according to claim 8, wherein the cancer is associated with BRAF^{V600X} mutations.

15. A combination of a BRAF inhibitor and a PD-1 axis binding antagonist for use according to claim 8, wherein the cancer is BRAF^{V600X} mutation-positive unresectable or metastatic cancer.

16. A combination of a BRAF inhibitor and a PD-1 axis binding antagonist for use according to any one of claims 7 to 8, wherein BRAF^{V600X} mutation is determined using a method comprising (a) performing PCR or sequencing on nucleic acid (e.g., DNA) extracted from a sample of the patient's tumour tissue and/or body fluid; and (b) determining expression of BRAF^{V600} in the sample.

17. A combination of a BRAF inhibitor and a PD-1 axis binding antagonist for use according to any one of claims 7 to 8, comprising one or more additional anticancer agents selected from MEK inhibitors, MEK degraders, EGFR inhibitors, EGFR degraders, inhibitors of HER2 and/or HER3, degraders of HER2 and/or HER3, SHP2 inhibitors, SHP2 degraders, Axl inhibitors, Axl degraders, ALK inhibitors, ALK degraders, PI3K inhibitors, PI3K degraders, SOS1 inhibitors, SOS1 degraders, signal transduction pathway inhibitors, checkpoint inhibitors, modulators of the apoptosis pathway, cytotoxic chemotherapeutics, angiogenesis-targeted therapies, immune-targeted agents, and antibody-drug conjugates.

18. A combination of a BRAF inhibitor and a PD-1 axis binding antagonist for use according to any one of claims 7 to 8, comprising in addition a MEK inhibitor.

19. A combination of a BRAF inhibitor and a PD-1 axis binding antagonist for use according to any one of claims 7 to 8, comprising an additional anticancer agent selected from EGFR inhibitors.

## Patentansprüche

1. Kombination eines BRAF-Inhibitors mit einem PD-1 achsenbindenden Antagonisten, wobei der BRAF-Inhibitor eine Verbindung der Formel (I) 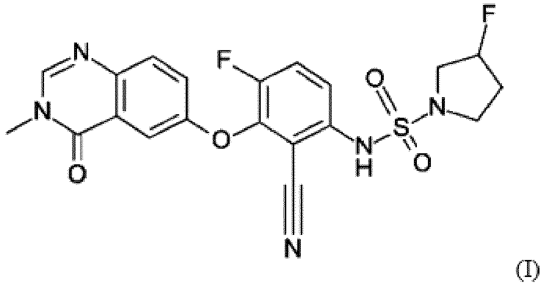 oder ein pharmazeutisch unbedenkliches Salz oder Solvat davon ist.

2. Kombination eines BRAF-Inhibitors mit einem PD-1 achsenbindenden Antagonisten nach Anspruch 1, wobei die Verbindung der Formel (I) (3R)-N-[2-Cyano-4-fluor-3-(3-methyl-4-oxochinazolin-6-yl)oxyphenyl]-3-fluorpyrrolidin-1-sulfonamid ist.

3. Kombination eines BRAF-Inhibitors mit einem PD-1 achsenbindenden Antagonisten nach Anspruch 1 oder 2, wobei der PD-1 achsenbindende Antagonist aus der Gruppe ausgewählt ist, die aus einem PD-1 bindenden Antagonisten, einem PD-L1 bindenden Antagonisten und einem PD-L2 bindenden Antagonisten besteht.

4. Kombination eines BRAF-Inhibitors mit einem PD-1 achsenbindenden Antagonisten nach einem der Ansprüche 1 bis 3, wobei der PD-1 achsenbindende Antagonist ein PD-1 bindender Antagonist ist.

5. Kombination eines BRAF-Inhibitors mit einem PD-1 achsenbindenden Antagonisten nach einem der Ansprüche 1 bis 3, wobei der PD-1 achsenbindende Antagonist aus Cemiplimab (Libtayo^{®}), Pembrolizumab (Keytruda^{®}), Nivolumab (Opdivo^{®}), RN888 (PF-06801591), Atezolizumab (Tecentriq^{®}), Avelumab (Bavencio^{®}) und Durvalumab (Imfinzi^{™}) ausgewählt ist.

6. Kombination eines BRAF-Inhibitors mit einem PD-1 achsenbindenden Antagonisten nach einem der Ansprüche 1 bis 4, wobei der PD-1 achsenbindende Antagonist Atezolizumab (Tecentriq^{®}) ist.

7. Kombination eines BRAF-Inhibitors mit einem PD-1 achsenbindenden Antagonisten nach einem der Ansprüche 1 bis 6 zur Verwendung als ein Medikament.

8. Kombination eines BRAF-Inhibitors mit einem PD-1 achsenbindenden Antagonisten nach einem der Ansprüche 1 bis 6 zur Verwendung bei der therapeutischen und/oder prophylaktischen Behandlung von Krebs.

9. Pharmazeutische Zusammensetzung, umfassend eine Kombination eines BRAF-Inhibitors mit einem PD-1 achsenbindenden Antagonisten nach einem der Ansprüche 1 bis 6 und einen oder mehrere pharmazeutisch unbedenkliche Hilfsstoff(e).

10. Kombination zur Verwendung nach einem der Ansprüche 7 bis 8, wobei der BRAF-Inhibitor oral verabreicht wird und der PD-1 achsenbindende Antagonist durch Injektion, wie intravenöse oder subkutane Injektion, verabreicht wird.

11. Kombination zur Verwendung nach einem der Ansprüche 7 bis 8 oder pharmazeutische Zusammensetzung nach Anspruch 9, wobei der BRAF-Inhibitor zusammen mit dem PD-1 achsenbindenden Antagonisten verabreicht wird.

12. Kombination zur Verwendung nach einem der Ansprüche 7 bis 8, wobei der BRAF-Inhibitor nacheinander mit dem PD-1 achsenbindenden Antagonisten verabreicht wird.

13. Kombination eines BRAF-Inhibitors mit einem PD-1 achsenbindenden Antagonisten zur Verwendung nach Anspruch 8, wobei der Krebs Schilddrüsenkrebs, Dickdarmkrebs, Melanom, Hirnkrebs, leptomeningealer Krebs oder nichtkleinzelliger Lungenkrebs ist.

14. Kombination eines BRAF-Inhibitors mit einem PD-1 achsenbindenden Antagonisten zur Verwendung nach Anspruch 8, wobei der Krebs mit BRAF^{V600X}-Mutationen in Verbindung steht.

15. Kombination eines BRAF-Inhibitors mit einem PD-1 achsenbindenden Antagonisten zur Verwendung nach Anspruch 8, wobei der Krebs BRAF^{V600X}-Mutation-positiver nicht resezierbarer oder metastatischer Krebs ist.

16. Kombination eines BRAF-Inhibitors mit einem PD-1 achsenbindenden Antagonisten zur Verwendung nach einem der Ansprüche 7 bis 8, wobei die BRAF^{V600X}-Mutation unter Anwendung eines Verfahrens bestimmt wird, das (a) Durchführen von PCR an oder Sequenzieren von Nukleinsäure (z. B. DNA), die aus einer Probe des Tumorgewebes und/oder der Körperflüssigkeit des Patienten extrahiert wurde; und (b) Bestimmen der Expression von BRAF^{V600} in der Probe umfasst.

17. Kombination eines BRAF-Inhibitors mit einem PD-1 achsenbindenden Antagonisten zur Verwendung nach einem der Ansprüche 7 bis 8, umfassend ein zusätzliches oder mehrere zusätzliche Antikrebsmittel, ausgewählt aus MEK-Inhibitoren, MEK-Abbauern, EGFR-Inhibitoren, EGFR-Abbauern, Inhibitoren von HER2 und/oder HER3, Abbauern von HER2 und/oder HER3, SHP2-Inhibitoren, SHP2-Abbauern, Axl-Inhibitoren, Axl-Abbauern, ALK-Inhibitoren, ALK-Abbauern, PI3K-Inhibitoren, PI3K-Abbauern, SOS1-Inhibitoren, SOS1-Abbauern, Signaltransduktionsweginhibitoren, Checkpoint-Inhibitoren, Modulatoren des Apoptoseweges, zytotoxischen Chemotherapeutika, gegen Angiogenese gerichteten Therapien, auf das Immunsystem gerichteten Mitteln und Antikörper-Arzneimittel-Konjugaten.

18. Kombination eines BRAF-Inhibitors mit einem PD-1 achsenbindenden Antagonisten zur Verwendung nach einem der Ansprüche 7 bis 8, die zusätzlich einen MEK-Inhibitor umfasst.

19. Kombination eines BRAF-Inhibitors mit einem PD-1 achsenbindenden Antagonisten zur Verwendung nach einem der Ansprüche 7 bis 8, die ein zusätzliches Antikrebsmittel umfasst, das aus EGFR-Inhibitoren ausgewählt ist.

## Revendications

1. Combinaison d'un inhibiteur de BRAF avec un antagoniste se liant à l'axe PD-1 dans laquelle l'inhibiteur de BRAF est un composé de formule (I) 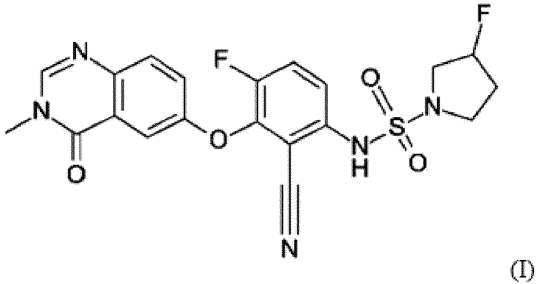 ou un sel pharmaceutiquement acceptable ou un solvate de celui-ci.

2. Combinaison d'un inhibiteur de BRAF avec un antagoniste se liant à l'axe PD-1 selon la revendication 1, dans laquelle le composé de formule (I) est le (3R)-N-[2-cyano-4-fluoro-3-(3-méthyl-4-oxo-quinazolin-6-yl)oxy-phényl]-3-fluoro-pyrrolidine-1-sulfonamide.

3. Combinaison d'un inhibiteur de BRAF avec un antagoniste se liant à l'axe PD-1 selon la revendication 1 ou 2, dans laquelle l'antagoniste se liant à l'axe PD-1 est choisi dans le groupe constitué par un antagoniste se liant à PD-1, un antagoniste se liant à PD-L1 et un antagoniste se liant à PD-L2.

4. Combinaison d'un inhibiteur de BRAF avec un antagoniste se liant à l'axe PD-1 selon l'une quelconque des revendications 1 à 3, dans laquelle l'antagoniste se liant à l'axe PD-1 est un antagoniste se liant à PD-1.

5. Combinaison d'un inhibiteur de BRAF avec un antagoniste se liant à l'axe PD-1 selon l'une quelconque des revendications 1 à 3, dans laquelle l'antagoniste se liant à l'axe PD-1 est choisi parmi le cémiplimab (Libtayo^{®}), le pembrolizumab (Keytruda^{®}), le nivolumab (Opdivo^{®}), le RN888 (PF-06801591), l'atézolizumab (Tecentriq^{®}), l'avélumab (Bavencio^{®}) et le durvalumab (Imfinzi^{™}).

6. Combinaison d'un inhibiteur de BRAF avec un antagoniste se liant à l'axe PD-1 selon l'une quelconque des revendications 1 à 4, dans laquelle l'antagoniste se liant à l'axe PD-1 est l'atézolizumab (Tecentriq^{®}).

7. Combinaison d'un inhibiteur de BRAF avec un antagoniste se liant à l'axe PD-1 selon l'une quelconque des revendications 1 à 6, pour une utilisation comme médicament.

8. Combinaison d'un inhibiteur de BRAF avec un antagoniste se liant à l'axe PD-1 selon l'une quelconque des revendications 1 à 6, pour une utilisation dans le traitement thérapeutique et/ou prophylactique du cancer.

9. Composition pharmaceutique comprenant une combinaison d'un inhibiteur de BRAF avec un antagoniste se liant à l'axe PD-1 selon l'une quelconque des revendications 1 à 6 et un ou plusieurs excipients pharmaceutiquement acceptables.

10. Combinaison pour une utilisation selon l'une quelconque des revendications 7 à 8, dans laquelle l'inhibiteur de BRAF est administré par voie orale et l'antagoniste se liant à l'axe PD-1 est administré par injection, telle qu'une injection intraveineuse ou sous-cutanée.

11. Combinaison pour une utilisation selon l'une quelconque des revendications 7 à 8 ou composition pharmaceutique selon la revendication 9, dans laquelle l'inhibiteur de BRAF est administré de manière simultanée avec l'antagoniste se liant à l'axe PD-1.

12. Combinaison pour une utilisation selon l'une quelconque des revendications 7 à 8, dans laquelle l'inhibiteur de BRAF est administré séquentiellement à l'antagoniste se liant à l'axe PD-1.

13. Combinaison d'un inhibiteur de BRAF avec un antagoniste se liant à l'axe PD-1 pour une utilisation selon la revendication 8, dans laquelle le cancer est un cancer de la thyroïde, un cancer colorectal, un mélanome, un cancer du cerveau, un cancer leptoméningé ou un cancer du poumon non à petites cellules.

14. Combinaison d'un inhibiteur de BRAF avec un antagoniste se liant à l'axe PD-1 pour une utilisation selon la revendication 8, dans laquelle le cancer est associé à des mutations BRAF^{V600X}.

15. Combinaison d'un inhibiteur de BRAF avec un antagoniste se liant à l'axe PD-1 pour une utilisation selon la revendication 8, dans laquelle le cancer est un cancer non résécable ou métastatique positif aux mutations BRAF^{V600X.}

16. Combinaison d'un inhibiteur de BRAF avec un antagoniste se liant à l'axe PD-1 pour une utilisation selon l'une quelconque des revendications 7 à 8, dans laquelle la mutation BRAF^{V600X} est déterminée en utilisant une méthode comprenant (a) la réalisation d'une PCR ou d'un séquençage sur l'acide nucléique (p. ex. l'ADN) extrait d'un échantillon d'un tissu tumoral et/ou d'un fluide corporel du patient; et (b) la détermination de l'expression de BRAF^{V600} dans l'échantillon.

17. Combinaison d'un inhibiteur de BRAF avec un antagoniste se liant à l'axe PD-1 pour une utilisation selon l'une quelconque des revendications 7 à 8, comprenant un ou plusieurs agents anticancéreux supplémentaires choisis parmi les inhibiteurs de MEK, les agents de dégradation de MEK, les inhibiteurs d'EGFR, les agents de dégradation d'EGFR, les inhibiteurs de HER2 et/ou HER3, les agents de dégradation de HER2 et/ou HER3, les inhibiteurs de SHP2, les agents de dégradation de SHP2, les inhibiteurs d'Axl, les agents de dégradation d'Axl, les inhibiteurs d'ALK, les agents de dégradation d'ALK, les inhibiteurs de PI3K, les agents de dégradation de PI3K, les inhibiteurs de SOS1, les agents de dégradation de SOS1, les inhibiteurs de voie de transduction du signal, les inhibiteurs de point de contrôle, les modulateurs de voie de l'apoptose, les agents chimiothérapeutiques cytotoxiques, les thérapies ciblant l'angiogenèse, les agents ciblant le système immunitaire et les conjugués anticorps-médicament.

18. Combinaison d'un inhibiteur de BRAF avec un antagoniste se liant à l'axe PD-1 pour une utilisation selon l'une quelconque des revendications 7 à 8, comprenant en outre un inhibiteur de MEK.

19. Combinaison d'un inhibiteur de BRAF avec un antagoniste se liant à l'axe PD-1 pour une utilisation selon l'une quelconque des revendications 7 à 8, comprenant un agent anticancéreux supplémentaire choisi parmi les inhibiteurs d'EGFR.
